# EUROPEAN PATENT SPECIFICATION

(11) **EP 2 046 982 B1**
(45) Date of publication and mention of the grant of the patent: **31.08.2011**
(21) Application number: 07784579.0
(22) Date of filing: 05.07.2007
(51) Int. Cl.: C12Q 1/68

(54) **IDENTIFICATION OF PATHOGENS**
IDENTIFIZIERUNG VON KRANKHEITSERREGERN
IDENTIFICATION DE PATHOGÈNES

(30) Priority: 05.07.2006 AT 11482006
(43) Date of publication of application: 15.04.2009
(73) Proprietor: AIT Austrian Institute of Technology GmbH, 1220 Wien (AT)
(72) Inventor: WIESINGER-MAYR, Herbert, 1120 Vienna (AT); PICHLER, Rudolf, 2485 Wampersdorf (AT); BODROSSY, Levente, 9086 Töltestava (HU); NÖHAMMER, Christa, 1100 Vienna (AT)
(74) Representative: Sonn & Partner Patentanwälte
(86) International application number: PCT/AT2007/000341
(87) International publication number: WO 2008/003114

(56) References cited:
- LIU YI ET AL: "Development and evaluation of 16S rDNA microarray for detecting bacterial pathogens in cerebrospinal fluid" EXPERIMENTAL BIOLOGY AND MEDICINE (MAYWOOD), vol. 230, no. 8, September 2005 (2005-09), pages 587-591, XP002450985 ISSN: 1535-3702
- KERAMAS G ET AL: "Development of a sensitive DNA microarray suitable for rapid detection of Campylobacter spp" MOLECULAR AND CELLULAR PROBES, ACADEMIC PRESS, LONDON, GB, vol. 17, no. 4, August 2003 (2003-08), pages 187-196, XP004450213 ISSN: 0890-8508
- WIESINGER-MAYR HERBERT ET AL: "Identification of human pathogens isolated from blood using microarray hybridisation and signal pattern recognition", BMC MICROBIOLOGY, BIOMED CENTRAL, LONDON, GB LNKD- DOI:10.1186/1471-2180-7-78, vol. 7, no. 1, 14 August 2007 (2007-08-14) , page 78, XP021028198, ISSN: 1471-2180
- WIESINGER-MAYR HERBERT ET AL: "Identification of human pathogens isolated from blood using microarray hybridisation and signal pattern recognition" BMC MICROBIOLOGY, BIOMED CENTRAL, LONDON, GB LNKD- DOI:10.1186/1471-2180-7-78, vol. 7, no. 1, 14 August 2007 (2007-08-14) , page 78, XP021028198 ISSN: 1471-2180

## Description

The present invention relates to the identification of pathogens of body fluid infections.

Despite continued progresses in diagnosis and early therapy of blood born infections mortality rates remain high. Traditional methods for the identification of microorganisms are based on blood culture methods requiring the microbial cultivation with subsequent morphological and physiological characterization (Peters et al., 2004).

The frequency of human pathogen occurrence has been periodically monitored by different scientists and several clinical research programs. It was shown that more than 95% of all bloodstream infections are caused by only 15 different genera. Staphylococcus sp. and Escherichia sp. account for more than 50% of the infections. Diversity studies varied only slightly between different countries and laboratories. While overall pathogen infection rates are stable over time, especially Pseudomonas aeruginosa infections are clearly increasing, representing the only pathogen associated with increased mortality rates (Fluit et al., 2001; Kempf et al., 2000; Shigei et al., 1995; Meremikwu et al., 2005; Vincent et al., 2006).

The first methods for bacterial quantity determination in bloodstream infections were based on spreading of whole blood on solid culture medium, incubation and subsequent evaluation by counting the colony forming units (CFU). Cultures isolated from patients with staphylococcal and streptococcal infections contained up to 100 CFU per ml blood, whereas E. coli bacteria were counted in excess of 1000 CFU/ml. Similar quantities were found for other gram negative bacteria (Yagupsky et al., 1990; Henry et al., 1983).

Recent publications based on molecular techniques proposed that the bacterial count may be higher than initially assumed. Quantitative RT-PCR was used to primarily define standard curves of bacterial quantities in whole blood for a subsequent determination of bacterial loads in clinical samples. The densities in blood were found to range from 10⁴ to 5.4x10⁵ bacteria per ml for Streptococcus pneumoniae. Other gram positive or negative microorganisms were detected at an extent of 10⁴ to 10⁷ per ml in bacteraemia patients. Hackett even showed a concentration peak in severe cases of septicaemia to a maximum of 1.8x10⁹ bacteria per ml (Hackett et al., 2002; van Haeften et al., 2003; Massi et al., 2005,). An explanation for the discrepancy between cultivation and molecular methods is the inability of some microorganisms to multiply under standard cultivation conditions (Keer and Birch, 2003). Furthermore methods based on DNA detection also include the non digested genomes of dead or static bacteria (Nogva et al., 2000; Nikkari et al., 2001).

Automated blood culture systems such as BacT/Alert and BAC-TEC9240 are the standard cultivation techniques in modern clinical practice. Several investigations have shown that false negative results occur periodically due to inappropriate growth conditions. Blood cultures without detectable microbial growth were further treated and subsequent positive results were obtained in 3 to 40% of the cases depending on the detection method (Shigei et al., 1995; Kocoglu et al., 2005; Karahan et al., 2006). Heininger et al. (1999) demonstrated the advantage of PCR detection of preceding antibiotic treatment in a rat model. The detection rate of classical blood cultures fell to 10% within 25 min after intravenous administration of cefotaxime, whereas the PCR detection rate was still 100% at that time.
Cultivation of yeasts is routinely carried out in special culture bottles. The offered systems perform at a sensitivity of 100% when used for the detection of Candida infections (Horvath et al., 2004).

Conventional diagnostic methods last at least 24 hours due to their requirement for microbial growth. In general the detection and identification is a lengthy process, usually ranging from 2 to 5 days for most organisms or even longer for fastidious organisms (Marlowe et al., 2003; Reimer et al., 1997; Henry et al., 1983). In contrast to this, DNA-based methods meet the need for a fast, reliable and thereby life-saving diagnosis (Belgrader et al., 1999; Vincent and Abraham 2006). However, these methods have not been able to adapt to the needs of specificity and sensitivity for the present field of blood diagnosis.

Rivers et al. (2005) highlighted the importance of early treatment within six hours after the first symptoms of bacteraemia in an intensive care unit (ICU), thus before the transition from sepsis to severe sepsis. It is expected that molecular assays will replace current conventional microbiological techniques for detection of bloodstream infections. Methods based on PCR amplification and subsequent hybridization of fluorescent probes seem to be the most promising approaches (Peters et al., 2004). Different molecular methods, including the utilization of fluorescently labelled probes, have been adapted for the detection of clinical pathogens. Fluorescent in situ hybridisation (FISH), PCR, Real time PCR, fluorescence-based PCR-single strand conformation polymorphism (SSCP), and oligonucleotide microarrays have been employed for the identification of microorganisms from bacteraemia patients however still including a cultivation-based bacterial enrichment step (Kempf V.A.J. et al., (2000); Peters et al., 2006; Mothershed E.A. and Whitney A.M. (2006); Rantakokko-Jalava (2000); Turenne C.Y. et al., (2000); Aoki S. et al., (2003); Martineau F. et al., (2001); Yadaf A.K. et al., (2005); Lehner A. et al., (2005); Shang S., et al., (2005)).

Microarray technology has been described as a powerful tool for various clinical applications such as pathogen identification of urinary tract infections (UTI), acute upper respiratory tract infections, periodontal pathogens and human intestinal bacteria. Microarrays are further applied for the analysis of microbial gene expression and diversity (Bryant et al., 2004; Kato-Maeda et al., 2001; Wang et al., 2002; Roth et al., 2004; Yu et al., 2004).

The WO 2001/07648 A1 describes a method for the identification with an amplification procedure such as PCR. Microorgansims can be categorized by the lengths of the amplificate.

The US 2004/0023209 A1 describes a primer extension reaction to visualize sequences of microorganisms for their identification. 16S and 18S rRNA can be used as probes.

According to the DE 197 13 556 A1 microorgansims can be identified by the distribution of short oligonucleotides. Specific distribution patterns can be associated to certain microorganisms like E. coli, B. subtilis and H. influenzae.

Liu et al. (Experimental Biology and Medicine, 230 (8) (2005): 587-591) describe a microarray with 16S rRNA specific probes for the identification of microorganisms in CSF. The oligonucleotide probes described therein should hybridise to only one bacterial species with minimised unspecific hybridisation.

Keramas et al. (Molecular and cellular Probes, 17 (4)(2003): 187-196) describe a DNA microarray for the identification of Campylobacter and other reference bacteria such as Arcobacter, Helicobacter and E. coli. The sample material used therein was chicken faecal material that might contain blood in the case of a Campylobacter infection.

In summary, traditional identification methods for microorganisms in everyday clinical life are usually based on time consuming cultivation with subsequent morphological and physiological characterization. Blood culture methods are the gold standard in the diagnosis of blood born microbial infections. However, early identification of infection causing microbes is the crucial requisite for a fast and optimally targeted infection treatment. However, unfortunately these conventional diagnostic methods last at least 24 hours due to their requirement for microbial enrichment.

It is therefore an object of the present invention to provide a fast but nevertheless reliable testing for pathogens in body fluids, especially those pathogens being related to or connected (or postulated to be connected) to human sepsis. Moreover a method is needed which is able to distinguish - also preferably on a fast track - between closely related, but pathologically or physiologically different species or types of organisms.

Accordingly, the present invention provides a method for identification of microbial pathogens, in particular infectious pathogens, in a body fluid sample comprising the following steps:
a) providing a body fluid sample (which is suspected to contain such microbial pathogens),
b) lysing the microbial pathogens (if present) and performing a nucleic acid amplification reaction on the microbial DNA encoding 16S or 18S rRNA wherein or whereafter the amplified nucleic acids are labelled,
c) contacting the labelled amplified nucleic acids of step b) with a microarray comprising on defined areas on the microarray's surface immobilised multispecific probes for microbial DNA encoding 16S or 18S rRNA from microbial pathogens,
d) detecting the binding of one or more species of the labelled amplified nucleic acids to a probe by detecting a labelled amplified nucleic acid being specifically bound to the microarray, and
e) identifying a microbial pathogen in the body fluid sample by correlating the detected binding of the labelled amplified nucleic acids with the defined areas of the immobilised multispecific probes for microbial DNA encoding 16S or 18S rRNA from microbial pathogens, comprising using the information of binding of labelled nucleic acids to multispecific probes immobilised on the microarray's surface by using predicted hybdridisation patterns with weighted mismatches.

In particular embodiments the microbial pathogen is of a blood stream infection, e.g. sepsis, and the body fluid sample is a blood sample. Thus a method for identification of microbial pathogens of bloodstream infections in a blood sample is provided comprising the following steps:
a) providing a blood sample (which is suspected to contain such microbial pathogens),
b) lysing the microbial pathogens (if present) and performing a nucleic acid amplification reaction on the microbial DNA encoding 16S or 18S rRNA wherein or whereafter the amplified nucleic acids are labelled,
c) contacting the labelled amplified nucleic acids of step b) with a microarray comprising on defined areas on the microarray's surface immobilised multispecific probes for microbial DNA encoding 16S or 18S rRNA from microbial pathogens of bloodstream infections,
d) detecting the binding of one or more species of the labelled amplified nucleic acids to a probe by detecting a labelled amplified nucleic acid being specifically bound to the microarray, and
e) identifying a microbial pathogen of bloodstream infections in the blood sample by correlating the detected binding of the labelled amplified nucleic acids with the defined areas of the immobilised multispecific probes for microbial DNA encoding 16S or 18S rRNA from microbial pathogens of bloodstream infections, comprising using the information of binding of labelled nucleic acids to multispecific probes immobilised on the microarray's surface by using predicted hybdridisation patterns with weighted mismatches.

In other preferred embodiments the pathogen is a vaginosis pathogen and the body fluid is vaginal fluid. Thus a method for identification of microbial pathogens of vaginosis in a vaginal fluid sample is provided comprising the following steps:
a) providing a sample of vaginal fluid (which is suspected to contain such microbial pathogens),
b) lysing the microbial pathogens (if present) and performing a nucleic acid amplification reaction on the microbial DNA encoding 16S or 18S rRNA wherein or whereafter the amplified nucleic acids are labelled,
c) contacting the labelled amplified nucleic acids of step b) with a microarray comprising on defined areas on the microarray's surface immobilised multispecific probes for microbial DNA encoding 16S or 18S rRNA from microbial pathogens of bloodstream infections,
d) detecting the binding of one or more species of the labelled amplified nucleic acids to a probe by detecting a labelled amplified nucleic acid being specifically bound to the microarray, and
e) identifying a microbial pathogen of vaginosis in the sample of vaginal fluid by correlating the detected binding of the labelled amplified nucleic acids with the defined areas of the immobilised multispecific probes for microbial DNA encoding 16S or 18S rRNA from microbial pathogens of vaginosis, comprising using the information of binding of labelled nucleic acids to multispecific probes immobilised on the microarray's surface by using predicted hybdridisation patterns with weighted mismatches.

With the present invention, a molecular approach is presented for the rapid identification of infectious pathogens, in blood combining nucleic acid amplification with microarray detection. The DNA chip according to the present invention comprises oligonucleotide capture probes for the relevant pathogens of human body fluids, for example, as provided in the example section as fully developed industrially applicable microchip 25 different pathogens including gram positive cocci, different genera of the Enterobacteriaceae family, non-fermenter and clinical relevant Candida species.

By using the microarray according to the present invention detection of microorganisms is possible within a short time frame, e.g. within 6 hours, enabling rapid diagnosis of pathogens from body fluids of infected patients at genus and species level and providing important conclusions for antibiotic treatments. Rapid diagnosis of bacterial infection speeds up the treatment and reduces healthcare. The sensitivity of the method is high and has been shown to be decreased to 10 bacteria per ml of whole blood depending on the infectious species, in the case of blood stream infectious pathogens.

Preferably, the nucleic acid amplification reaction on the microbial DNA encoding 16S or 18S rRNA is performed by a PCR reaction. The amplification reaction can be performed by e.g. Multiplex-PCR, however, according to the present invention reduction in primer number for the nucleic acid amplification has proven to be advantageous. Therefore, in the method according to the present invention the nucleic acid amplification reaction on the microbial DNA encoding 16S or 18S rRNA is preferably performed with universal primers for the microbial DNA encoding 16S or 18S rRNA, preferably with not more than eight (4 forward, 4 reverse) primers, more preferred with not more than six (3 forward, 3 reverse) primers, preferably with not more than four (2 forward, 2 reverse) primers. The primers according to Seq.ID Nos. 1, 2, 4 and 5 have been identified as being specifically suitable for the present method.

As a blood sample, any sample from patients being suspected of having such bloodstream pathogens are usable including samples from processed blood preparations such as blood fractions, blood derivatives or blood products. According to the present invention it is specifically preferred to perform an initial filtration step before performing the nucleic acid amplification reaction wherein the body fluid sample, in particular the blood sample, is filtered through a filter withholding leukocytes present in said body fluid sample but not withholding the microbial pathogens. Usually, leukocytes have an exclusion size of 11µm (diameter) whereas most of the (bacterial) pathogens to be identified by the present invention have a size of 2 µm. Accordingly, for example a filter with an exclusion size of 5 to 10 µm, preferably of 7µm is absolutely suitable for this filtration step.

By far the largest field of application of the present method is the diagnostics of human blood sample, especially in connection with patients having sepsis or are at risk of developing sepsis. However, the present method is as suitable for testing of large series of samples, e.g. in testing of hospital personnel or veterinary testing (e.g. of a larger number of animals). Preferably, however, the testing according to the present method is performed on the identification of human pathogens.

For labelling of nucleic acids, especially DNA, during or after amplification many methods are available to the skilled man in the art. For example, the labelling of the nucleic acids is performed by primer extension, in vitro transcription, biotin-streptavidin-labelling, isothermal Klenow fragment based labelling or direct nucleic amplification labelling, preferably by direct PCR labelling. The most preferred labelling method according to the present invention is primer extension, preferably primer extension using fluorescence dyes, especially Cy5. This preferred embodiment showed the best sensitivity and specificity.

According to a preferred embodiment of the method according to the present invention the amplified labelled nucleic acids are directly applied to the microarray without a purification or washing step after the nucleic acid amplification reaction. Surprisingly, the non-purification did not lead to adverse effects during binding of the products to the microarray. In contrast, because of the lack of further purification of the nucleic acid before binding to the microarray, loss of products is prevented.

The method according to the present invention may comprise in its experimental procedure DNA isolation from blood, multiplex PCR, fluorescence labelling (Cy5-dCTP) by a primer extension step and subsequent microarray hybridization.

Preferably, the microarray according to the present invention comprises immobilised probes for microbial DNA encoding 16S or 18S rRNA from at least ten, preferably at least 15, especially at least 20, of the following microbial pathogens: Escherichia coli (ATCC 35218, EC5, EC17, 81617, 68933, 68307), Enterobacter aerogenes (DSMZ 30053, 12676), Enterobacter cloacae (26385, 79232, 93840, 12720, 74892), Klebsiella pneumoniae (25809, 85813, 26385, 13253), Klebsiella oxytoca (26785, 26384, 73739, 26786, 96633), Citrobacter koseri (DSMZ 4595), Citrobacter freundii (80324, 73489), Staphylococcus aureus (ATCC 6538, ATCC 25923, ATCC 29213, 83799, 82913, 73237, 12998), Staphylococcus epidermidis (ATCC 14990, 73711, 35989, 80320, 13000, 77504, 79510), Enterococcus faecalis (ATCC 29212, EF4, 81239, 83776, 27520), Enterococcus faecium (DSMZ 20477), Streptococcus pneumoniae (DSMZ 25500), Streptococcus pyogenes (ATCC 19615, 10388), Proteus mirabilis (26786, ATCC 14153, 27761, 97656, 71913), Proteus vulgaris (DSMZ 13387, 80196), Serratia marcescens (DSMZ 30121), Morganella morganii (DSMZ 6675, 12615), Pseudomonas aeruginosa (26178, 12950, 26535, 68961, 74352), Stenotrophomonas maltophilia (DSMZ 50170, 26394, 26396), Acinetobacter baumannii (DSMZ 30007), Acinetobacter lwoffii (DSMZ 2403, 75496), Acinetobacter radioresistens (DSMZ 6976), Acinetobacter johnsonii (DSMZ 6963), Candida albicans (ATCC 10231, 21179, 27184, 96917, 96635), Candida parapsilosis (4344). These pathogens are of particular importance in the case of blood stream infections.

According to a preferred embodiment, the microarray according to the present invention comprises at least one strain of at least 10 different species, preferably of at least 15 different species, especially of at least 20 different species, of the following species: Escherichia coli, Enterobacter aerogenes, Enterobacter cloacae, Klebsiella pneumoniae, Klebsiella oxytoca, Citrobacter koseri, Citrobacter freundii, Staphylococcus aureus, Staphylococcus epidermidis, Enterococcus faecalis, Enterococcus faecium, Streptococcus pneumoniae, Streptococcus pyogenes, Proteus mirabilis , Proteus vulgaris, Serratia marcescens, Morganella morganii, Pseudomonas aeruginosa, Stenotrophomonas maltophilia, Acinetobacter baumannii, Acinetobacter lwoffii, Acinetobacter radioresistens, Acinetobacter johnsonii, Candida albicans, Candida parapsilosis.

A preferred embodiment of the microarray according to the present invention comprises immobilised probes which are multispecific. Under "multispecific" according to the present invention a specificity in binding to more than one of the microbial pathogens possibly present in a body fluid sample is understood. This means that a specific binding of a single probe can be obtained for the amplified nucleic acids of more than one pathogen. However, identification of nucleic acid being specific for more than one Proteus type (e.g. mirabilis or vulgaris) or for more than one Acinetobacter type (e.g. baumannii, lwoffii, radioresistens, or johnsonii) is not regarded as "multispecific" according to the present invention, only e.g. a probe which specifically recognises Serratia marcescens and Citrobacter freundii, Pseudomonas aeruginosa and Stenotrophomonas maltophilia, or Escherichia coli, Proteus mirabilis and Serratia marcescens (yet each possibly with different intensities) will be regarded as "multispecific" according to the present invention.

The microarray according to the present invention preferably comprises the probes as spots on the surface, preferably in each of the spots only one species of probes is present. The probes of the present invention are nucleic acid molecules, especially DNA molecules which bind to nucleic acids amplified according to the present invention, i.e. specific for pathogen microbial DNA encoding 16S or 18S rRNA. Preferably the probe binds to the portion of the amplified nucleic acid which is located between the primer sequences of the amplification reaction, thereby amplifying only the amplified portion of the amplification product and not the primer sequences. With this embodiment, the risk of detecting false positive signals due to primer binding of the probe can be excluded.

Preferably, the microarray according to the present invention comprises at least 10, preferably at least 20, more preferred at least 30, especially at least 40 multispecific immobilised probes. According to a specific embodiment of the present invention, the microarray preferably comprises a portion of at least 20% multispecific probes, preferably at least 40% multispecific probes, especially at least 50% multispecific probes, of the total number of probes immobilised on the microarray.

A preferred microarray according to the present invention comprises at least 5, preferably at least 10, more preferred at least 20, even more preferred at least 30, especially at least 50, of the probes according to Seq.ID Nos 6 to 80. Preferably, the probes are selected to represent at least 80%, preferably at least 90%, more preferred at least 95%, especially at least 98%, of the microbial, especially bacterial, pathogens connected with or suspected of being connected with (by acknowledged medical authorities) sepsis on the microchip.

The correlation of step e) is performed by using the information of binding of labelled nucleic acids to multispecific probes immobilised on the microarray's surface. This correlation may be performed by computer analysis. For example, performing the correlation of step e) by using predicted hybridisation patterns with weighted mismatches has proven to deliver excellent results for the testing according to the present invention. A prototype software providing a statistical evaluation routine was developed, allowing correct identification in 100% of the cases at the genus and in 96% at the species level. This self learning software (as described in the example section of the present application) can be implemented in a fully automated analysis platform to be supplied with the pathogen identification microarray.

According to another aspect, the present invention relates to a kit comprising a microarray as defined above and a computer program product for identifying a microbial pathogen by correlating detected binding of labelled or amplified nucleic acids with defined areas of bound multispecific probes for microbial DNA encoding 16S or 18S rRNA from microbial pathogens on the microarray using the information of binding of labelled or amplified nucleic acids to multispecific probes immobilised on the microarray's surface by using predicted hybridisation patterns with weighted mismatches. A microarray (also commonly known as gene chip, DNA chip, or biochip) is a collection of microscopic DNA spots attached to a solid surface, such as glass, plastic or silicon chip forming an array for the purpose of expression profiling, monitoring levels for a large number of amplified nucleic acids simultaneously. Microarrays can be fabricated using a variety of technologies, including printing with fine-pointed pins onto glass slides, photolithography using pre-made masks, photolithography using dynamic micromirror devices, ink-jet printing, or electrochemistry on microelectrode arrays. A microarray comprises a large number of immobilized oligonucleotide molecules provided in high density on the solid support. A microarray is a highly efficient tool in order to detect dozens, hundreds or even thousands of different amplification products according to the present invention in one single detection step. Such microarrays are often provided as slides or plates in particular microtiter plates. In the state of the art a microarray is both defined either as a miniaturized arrangement of binding sites (i.e. a material, the support) or as a support comprising miniaturized binding sites (i.e. the array). Both definitions can be applied for the embodiment of the pre-sent invention. For the first of these definitions the preferred embodiment of the present invention is a miniaturized arrangement of the oligonucleotides of the present invention in a mi-croarray. The oligonucleotide molecules are preferably immobilised onto the microarray with the help of a printing device which ensures immobilization in high density on the solid sup-port. This microarray is particularly useful when analysing a large number of samples. The microarray according to the present invention is usually a flat surface with the probes immobilised in regular patterns over this surface at defined positions.

According to an alternative embodiment, the present invention provides a method for identification of microbial pathogens in a body fluid sample comprising the following steps:
a) providing a body fluid sample (which is suspected to contain such microbial pathogens),
b) lysing the microbial pathogens (if present) and performing a nucleic acid amplification reaction on the microbial DNA encoding 16S or 18S rRNA,
c) contacting the amplified nucleic acids of step b) with a microarray comprising on defined areas on the microarray's surface immobilised multispecific probes for microbial DNA encoding 16S or 18S rRNA from microbial pathogens,
d) detecting the binding of one or more species of the amplified nucleic acids to a probe by detecting a amplified nucleic acid being specifically bound to the microarray by a device of the microarray which detects the binding event of an amplified nucleic acid to an immobilised probe, and
e) identifying a microbial pathogen in the body fluid sample by correlating the detected binding of the amplified nucleic acids with the defined areas of the immobilised multispecific probes for microbial DNA encoding 16S or 18S rRNA from microbial pathogens, using the information of binding of amplified nucleic acids to multispecific probes immobilised on the microarray's surface by using predicted hybridisation patterns with weighted mismatches.

According to this specific alternative method according to the present invention, labelling of the amplified nucleic acids is not necessary, the binding event is detected by a hybridisation signal on the specific probe on the microarray. This can be arranged on the microarray according to conventional techniques available in the field, so that each probe or spot of probe can be analysed whether a specific binding (hybridisation) signal has taken place (or not). In this specific embodiment, the microarray according to the present invention comprises additional means or devices to detect a specific binding signal to a probe or a given area on the microarray's surface. These devices include interfaces to computers making the binding events visible on e.g. graphic representations so that binding events on the chip (microarray) can effectively correlated to give a reasonable analytical result under step e) according to the present invention.

In particular in the case of blood stream infections a method for identification of microbial pathogens of bloodstream infections in a blood sample is provided comprising the following steps:
a) providing a blood sample (which is suspected to contain such microbial pathogens),
b) lysing the microbial pathogens (if present) and performing a nucleic acid amplification reaction on the microbial DNA encoding 16S or 18S rRNA,
c) contacting the amplified nucleic acids of step b) with a microarray comprising on defined areas on the microarray's surface immobilised multispecific probes for microbial DNA encoding 16S or 18S rRNA from microbial pathogens of bloodstream infections,
d) detecting the binding of one or more species of the amplified nucleic acids to a probe by detecting a amplified nucleic acid being specifically bound to the microarray by a device of the microarray which detects the binding event of an amplified nucleic acid to an immobilised probe, and
e) identifying a microbial pathogen of bloodstream infections in the blood sample by correlating the detected binding of the amplified nucleic acids with the defined areas of the immobilised multispecific probes for microbial DNA encoding 16S or 18S rRNA from microbial pathogens of bloodstream infections, using the information of binding of amplified nucleic acids to multispecific probes immobilised on the microarray's surface by using predicted hybridisation patterns with weighted mismatches.

In a further aspect the present invention provides a the method of present invention provides a method for identification of microbial pathogens of vaginosis (also referred to as vaginitis) in a sample of vaginal fluid comprising the following steps:
a) providing a vaginal fluid sample (which is suspected to contain such microbial pathogens),
b) lysing the microbial pathogens (if present) and performing a nucleic acid amplification reaction on the microbial DNA encoding 16S or 18S rRNA,
c) contacting the amplified nucleic acids of step b) with a microarray comprising on defined areas on the microarray's surface immobilised multispecific probes for microbial DNA encoding 16S or 18S rRNA from microbial pathogens of vaginosis,
d) detecting the binding of one or more species of the amplified nucleic acids to a probe by detecting a amplified nucleic acid being specifically bound to the microarray by a device of the microarray which detects the binding event of an amplified nucleic acid to an immobilised probe, and
e) identifying a microbial pathogen of bloodstream infections in the blood sample by correlating the detected binding of the amplified nucleic acids with the defined areas of the immobilised multispecific probes for microbial DNA encoding 16S or 18S rRNA from microbial pathogens of vaginosis, using the information of binding of amplified nucleic acids to multispecific probes immobilised on the microarray's surface by using predicted hybridisation patterns with weighted mismatches.

Preferably the pathogen of vaginosis to be identified is selected from Gardnerella vaginalis, Atopobium, Mobiluncus and Bacteroides. In particular the immobilised probes is selected from SEQ ID NOs 81 to 138 of table 4 below.

A healthy vagina normally contains many microorganisms, some of the common ones are Lactobacillus crispatus and Lactobacillus jensenii. Lactobacillus, particularly hydrogen peroxide-producing species, appear to help prevent other vaginal microorganisms from multiplying to a level where they cause symptoms. The microorganisms involved in bacterial vaginosis are very diverse, but are always accompanied by one of the marker species Gardnerella vaginalis, Atopobium, Mobiluncus and Bacteroides. A change in normal bacterial flora including the reduction of lactobacillus, which may be due to the use of antibiotics or pH imbalance, allows more resistant bacteria to gain a foothold and multiply. In turn these produce toxins which effect the body's natural defense and make re-colonization of healthy bacteria more difficult.

The presence of the vaginosis marker species amongst other human pathogens can be detected by using a DNA microarray which consists of species specific as well as multi-specific probes leading to a characteristic signal pattern subsequent to hybridisation. The evaluation of hybridisation signal pattern based on the described statistical method allows a clear discrimination of the infecting species as well as the marker species. The creation of a database consisting of quantile normalised signal intensities and the statistical analysis of single hybridisations was realised as described herein (Sha et al. (2005) J.Clin.Microbiol., 43, 4607-4612, Donders et al. (1998) N. Engl. J. Med., 338, 1548, Donders (1999) Eur. J. Obstet. Gynecol. Reprod. Biol., 83, 1-4, Donders (1999) Infect. Dis. Obstet. Gynecol., 7, 126-127).

According to another embodiment, the present invention relates to a test kit comprising a sample holding means for a blood sample, a microarray according to the present invention and optionally primers to perform the amplification reaction according to the present invention. For example, the test kit according to the present invention may contain primers being specific for amplification of microbial DNA encoding 16S and 18S rRNA of the pathogens as defined above.

According to another embodiment, the present invention also relates to the use of a microarray according to the present invention or a test kit according to the present invention for the identification of microbial pathogens of bloodstream infections in a blood sample, especially for monitoring the blood of a sepsis patient or a patient being at risk of developing sepsis.

In a preferred embodiment of all aspects of the present invention, including the use of the microarray for the inventive method, the amplification, e.g. by PCR, and/or labelling, e.g. by primer extension, is performed with a polymerase selected from Thermus species (e.g. Thermus aquaticus, Thermus flavus or Thermus thermophilus) polymerases, e.g. Taq polymerase I, in particular GoTaq^{®} or FirePol^{®} DNA Polymerase. Particular exceptional results were achieved with these two optimized polymerases. FirePol is a thermostable polymerase and similar to Taq DNA polymerase I (homology 98%) with 3' to 5' exonuclease activity. Preferably the polymerase has increased temperature resistance compared to Taq polymerase I, preferably by at least 1°C, 2°C, 3°C, 4°C, 5°C or more, and/or has 3' to 5' exonuclease activity and/or lacks 5' to 3' exonuclease activity. Specific polymerases are e.g. described in the EP 0745676 A1 or US 5079352. The reaction is further preferably performed at a pH between 7 and 9, in particular preferred above 8, most preferred at about 8.5, e.g. 8.2 to 8.7. Mg, e.g. in form of MgCl₂, may be present for the polymerisation reaction, e.g. in a concentration of between 0.5 mM to 5 mM, preferably between 1 mM and 3 mM, most preferred about 1.5 mM.

Also disclosed is a method for the identifying pathogens comprising
a) providing a matrix of signal data of detected binding events of nucleotide material of the pathogen to probes specific for a pathogen
b) quantile normalizing the matrix,
c) classification of the signal data by the k-nearest neighbour (KNN) method.

Using the KNN algorithm the signal data is classified by a majority vote of its neighbours, with the signal being assigned the class most common amongst its k nearest neighbours as described by Ripley (1996) "Pattern Recognition and Neural Networks", Cambridge and Venables et al. (2002), "Modern Applied Statistic with S.", 4th Ed., Springer; Quantile Normalization was performed according to Bolstad et al., Bioinformatics 19(2) (2003), 185-193. Preferably k is 1 the signal is simply assigned the class of its nearest neighbour.

In particular the matrix comprises data of at least 2, 3, 4, 5, 6, 7, 8, 9, 10, 11, 12, 14, 16, 18, or 20 pathogens. Preferably for each pathogen to be detected at least 1 probe is used to generate a signal. However also more different probes for each pathogen can be used, e.g. 2, 3, 4, 5, 6, 7, 8, 10 or more. In other words at least two signal data of binding events is present in the matrix. In particular if more probes are used an the median of the signal data of the probes detected for each pathogen is used for the method, in particular for the step of classification. Preferably the classifier is validated in a step d) by a cross-validation method, in particular by the leave-one-out method. Cross-validation is the statistical practice of partitioning the data matrix into subsets such that the analysis is initially performed on a single subset, while the other subset(s) are retained for subsequent use in confirming and validating the initial analysis. The initial subset of the matrix is called the training set and the other subsets are called validation or testing sets. The leave-one out method involves using a single signal data from the matrix as the validation data, and the remaining signals as the training data. This is repeated such that each signal data in the sample is used once as the validation data.

Preferably the nucleotide material of the pathogen is DNA or RNA, in particular 16S rRNA or 18S rRNA.

Preferably the binding events includes data of multispecific probes which bind two or more pathogens, preferably pathogens of blood stream infections or pathogens of vaginal fluid.

The present invention is further illustrated by the following figures and examples without being restricted thereto.
Fig. 1 shows a phylogenetic tree based on 16S and 18S rRNA sequence analysis of, on the newly developed microarray represented, microorganisms calculated by the neighbour joining method.
Fig. 2 shows the matrix predicting hybridization behaviour of the designed microarray probes (horizontally plotted). Ranges of mismachtes are colour coded. The initial file comprised about 19.000 species. Fig. 2B shows the legend for Fig. 2: Colour key of weighted mismatches.

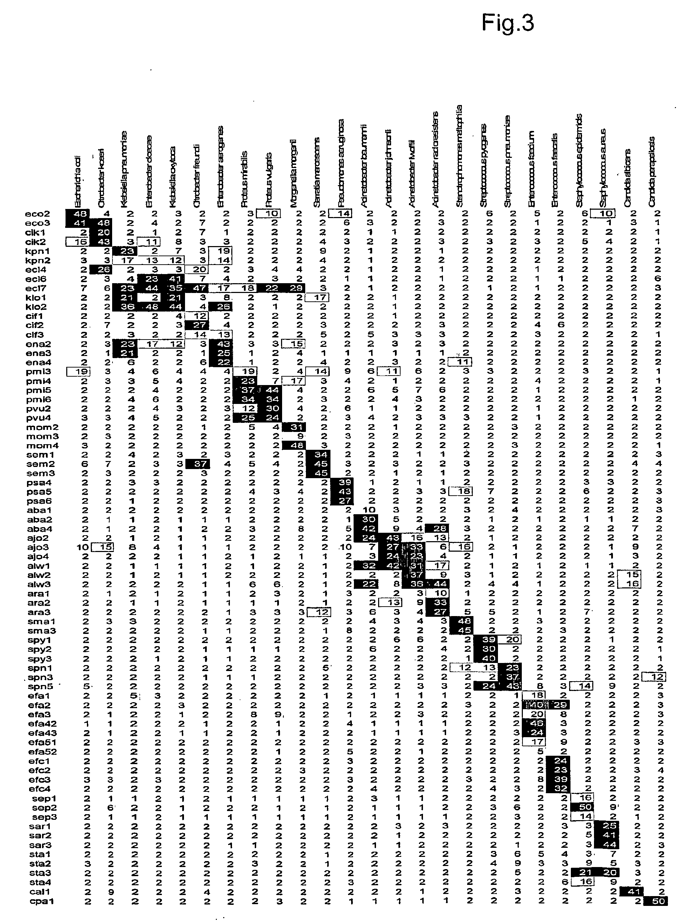

Fig. 3 shows normalized signal intensities of all hybridization experiments listed by probe and species. The raw signal values were first normalised using quantile normalization, and then averaged across spot-replicates and hybridization-replicates (real values were divided by 1000 for better visualization). Background corrected hybridization signals of 5001 - 10000, 10001 - 20000, and >20001, are indicated in yellow, orange and red, respectively. Normalized values lower than 5000 are not colour-coordinated. For calculations absolute values were used without defining a threshold that led to indication of low signals even when signals were flagged negative by the GenePix software. Species are listed according to the phylogenetic relation of 16S and 18S rRNA sequences. Probes are sorted by species specificity. Abbreviations of probe names are listed in table 3.
Fig. 4 shows PCR products of dilution series from bacterial cell cultures resolved on a 1.5% agarose gel. Bands can be detected from an initial count of 10³ bacteria per assay.
Fig. 5 shows graphs of the lowest dilution step in which a positive signal on the microarray could be detected. The dilution series was made of pure cultures from E. coli (Fig. 5A) and Staphylococcus aureus (Fig. 5B). E. coli shows a much lower detection limit of 10 bacteria per assay than Staphylococcus aureus with 10³ bacteria per assay. Red, blue and yellow bars represent specific and non-specific signals as well as positive controls (BSrev is the hybridization control and pr_FW and pr_FW T7 are PCR amplification controls). The labelled target derived from PCR product shown in fig. 4.
Fig. 6 shows a comparison of different parallel identification of pathogens. Heatmap was drawn after hierarchical clustering. Each target combination was compared with hybridization results of single cultures under equal experimental conditions. Rows correspond to probes and columns correspond to hybridizations. Colours correspond to signal values. So that blue displays high signal value and red no signal value.
Fig. 7 shows hybridization signals of E. coli isolated from whole blood. Despite the great background of human DNA in blood no interference (non-specific signals would be displayed blue) were observed. Specific signals are shown as red and positive controls as yellow bars.
Fig. 8 shows the isolation of bacterial DNA from blood spiked E.coli and Proteus mirabilis, simulating a multi-microbial infection. Abbreviations of probe names are listed in table 3. Red, blue and yellow bars represent specific and non-specific signals as well as positive controls
Fig. 9 shows the effects of quantile normalization.

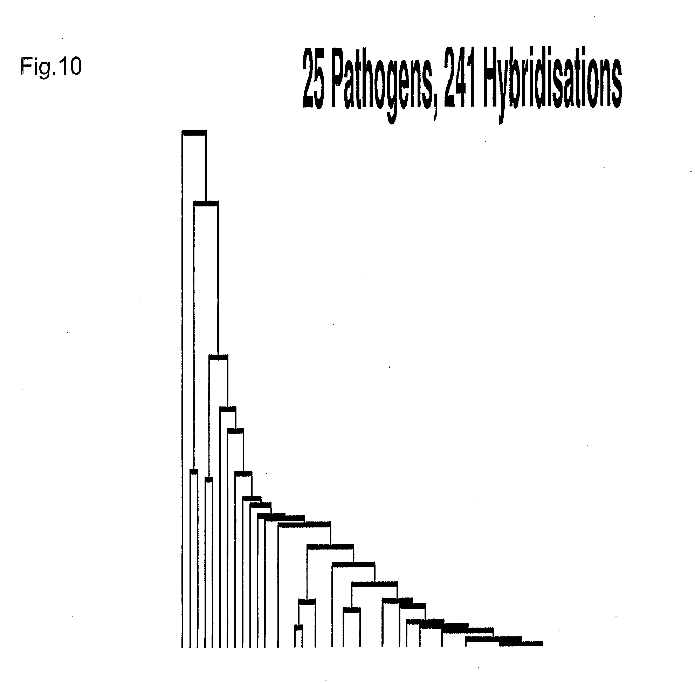

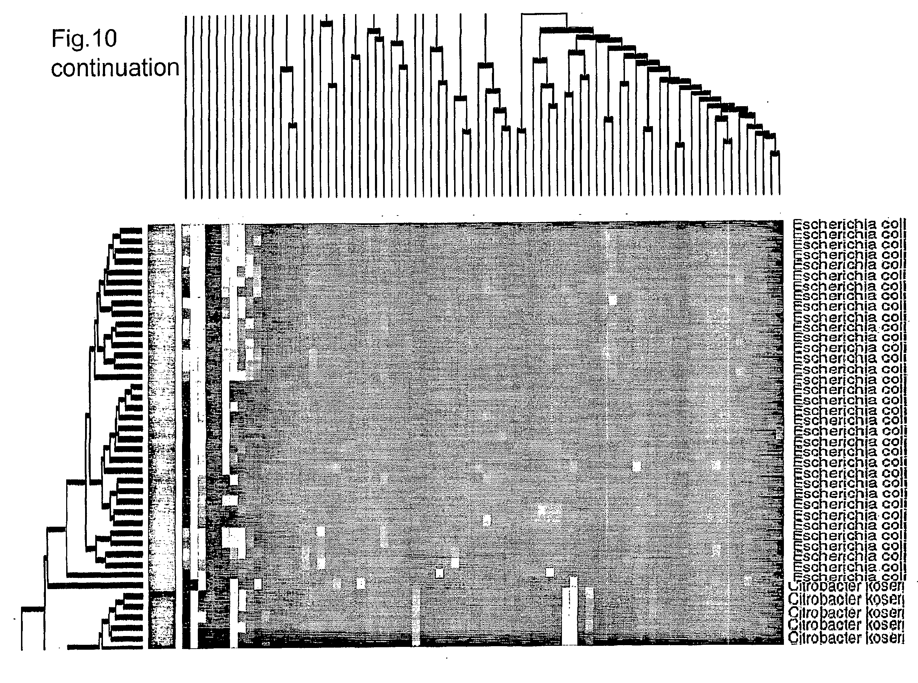

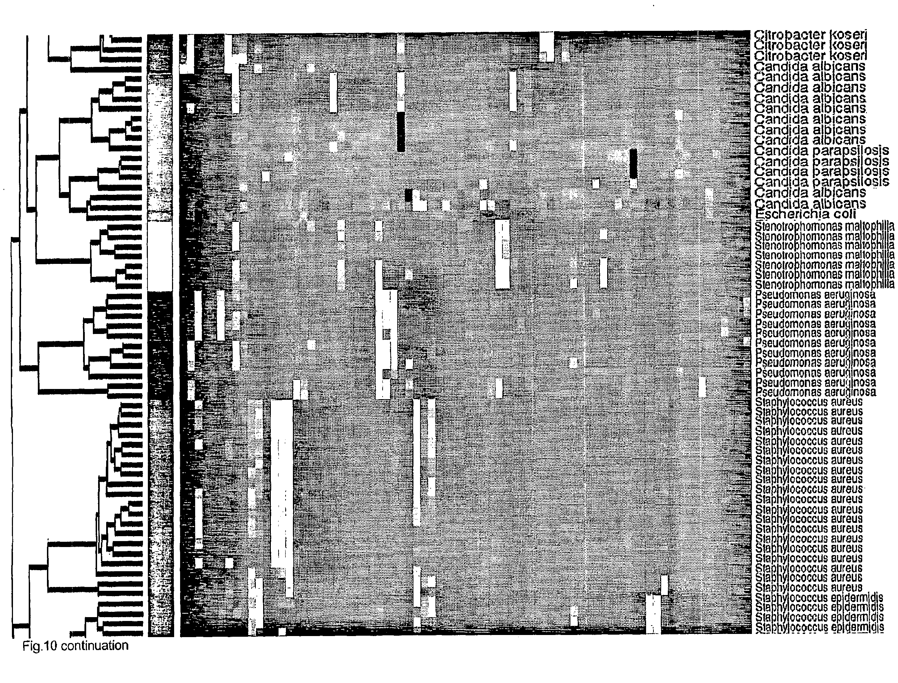

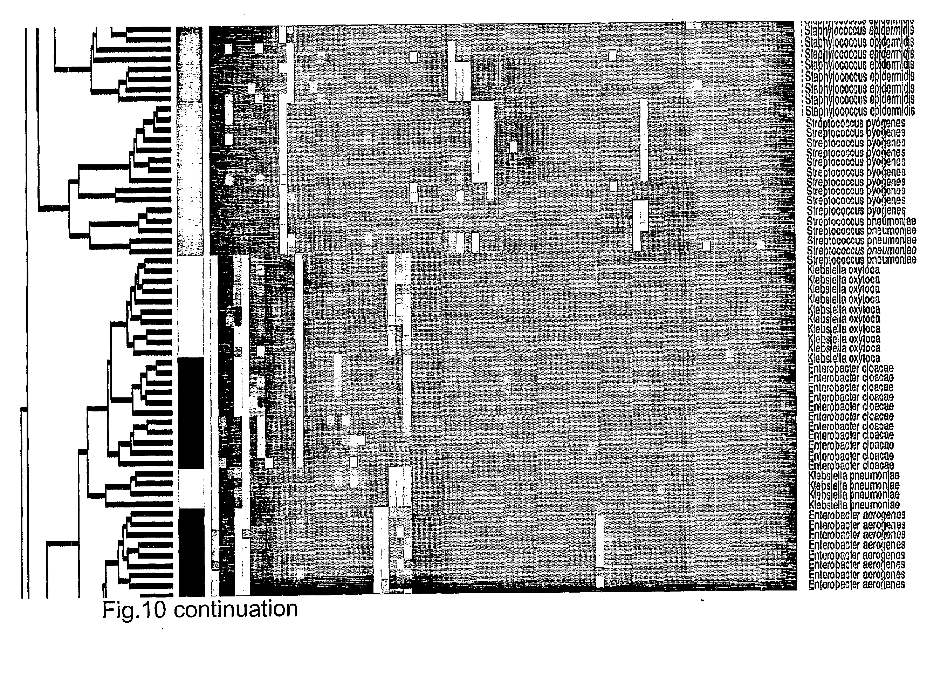

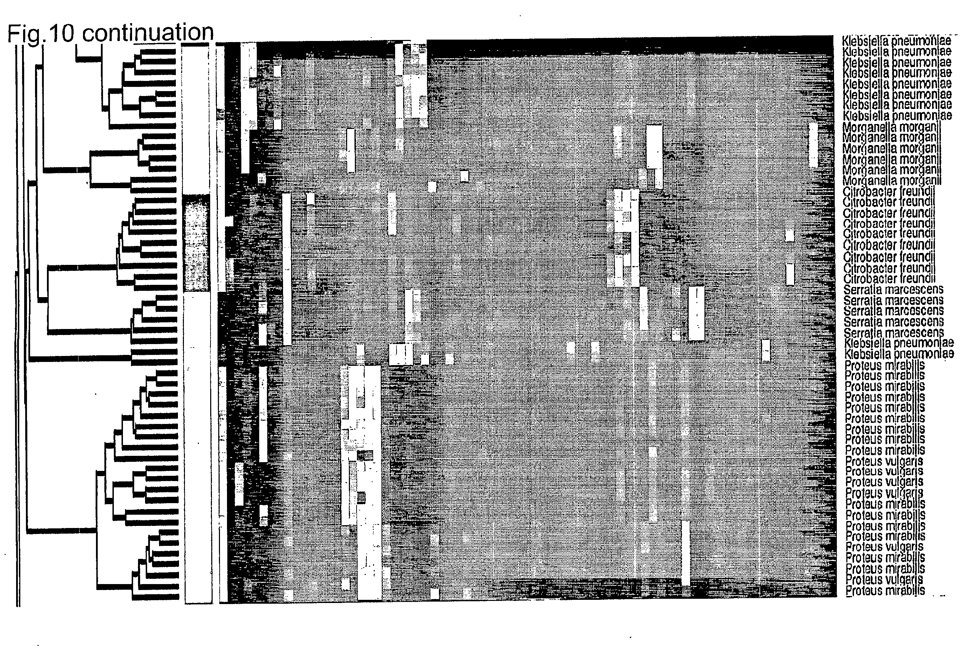

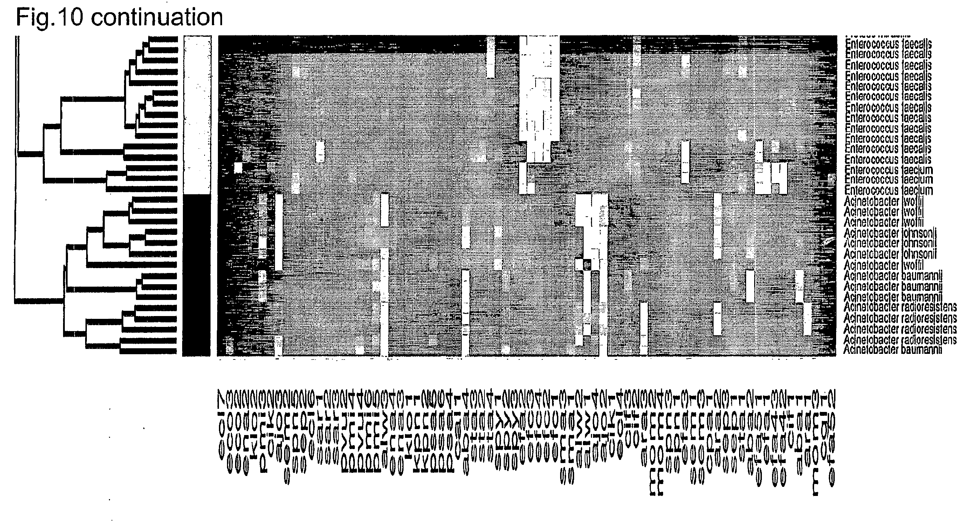

Fig. 10 shows the results of all hybridization experiments as a heatmap after hierarchical clustering. Columns correspond to probes and rows correspond to hybridizations. Colours correspond to signal values. The coefficient of variation of the different assays was already given along with the table of normalized signal values. One hybridization result with E. coli targets was clustered isolated from the others due to a false negative signal of the eco2 probe. However during identification procedures this was avoided by the rank transformation and k nearest neighbour method that still gave the correct result. The rows showing the hybridisations can be assigned to the microorganisms detected (from top to down): Escherichia coli (35 times), Citrobacter koseri (8 times), Candida albicans (8 times), Candida parapsilosis (4 times), Candida albicans (2 times), Escherichia coli (1 time), Stenotrophomona maltophila (7 times), Pseudomonas aeruginosa (11 times), Staphylococcus aureus (20 times), Staphylococcus epidermis (12 times), Streptococcus pyogenes (10 times), Streptococcus pneumoniae (5 times), Klebsiella oxytoca (10 times), Enterobacter cloacae (11 times), Klebsiella pneumoniae (4 times), Enterobacter aerogenes (11 times), Klebsiella pneumoniae (8 times), Morganella morganii (6 times), Citrobacter freundii (9 times), Serratia marcescens (5 times), Klebsiella pneumoniae (2 times), Proteus mirabilis (9 times), Proteus vulgaris (4 times), Proteus mirabilis (4 times), Proteus vulgaris (1 time), Proteus mirabilis (2 times), Proteus vulgaris (1 time), Proteus mirabilis (1 time), Enterococcus faecalis (12 times), Enterococcus faecium (3 times), Acinetobacter lwoffi (3 times), Acinetobacter johnsonii (3 times), Acinetobacter lwoffi (1 time), Acinetobacter baumannii (3 times), Acinetobacter radioresistens (4 times), Acinetobacter baumannii (1 time).

### EXAMPLES:

### Example 1: Samples - Reference Strains

All reference strains tested in this study were obtained from the American type culture collection (ATCC) or the "Deutsche Sammlung für Mikroorganismen und Zellkultur" (DSMZ). In addition to the reference strains probe specificity and sensitivity were also tested with clinical isolates which had been identified by classical microbiology methods. For long term storage all bacterial strains were kept as 50% glycerol stocks at -80°C. For most of the experiments pure cultures of a certain number of bacteria per ml were used which were obtained by cultivating the respective microbe in Caso bouillon overnight at 37°C and finally adjusting the microbe concentration per ml using a Mc Farland standard # 0,5. Microarray testing was performed on Escherichia coli (ATCC 35218, EC5, EC17, 81617, 68933, 68307), Enterobacter aerogenes (DSMZ 30053, 12676), Enterobacter cloacae (26385, 79232, 93840, 12720, 74892), Klebsiella pneumoniae (25809, 85813, 26385, 13253), Klebsiella oxytoca (26785, 26384, 73739, 26786, 96633), Citrobacter koseri (DSMZ 4595), Citrobacter freundii (80324, 73489), Staphylococcus aureus (ATCC 6538, ATCC 25923, ATCC 29213, 83799, 82913, 73237, 12998), Staphylococcus epidermidis (ATCC 14990, 73711, 35989, 80320, 13000, 77504, 79510), Enterococcus faecalis (ATCC 29212, EF4, 81239, 83776, 27520), Enterococcus faecium (DSMZ 20477), Streptococcus pneumoniae (DSMZ 25500), Streptococcus pyogenes (ATCC 19615, 10388), Proteus mirabilis (26786, ATCC 14153, 27761, 97656, 71913), Proteus vulgaris (DSMZ 13387, 80196), Serratia marcescens (DSMZ 30121), Morganella morganii (DSMZ 6675, 12615), Pseudomonas aeruginosa (26178, 12950, 26535, 68961, 74352), Stenotrophomonas maltophilia (DSMZ 50170, 26394, 26396), Acinetobacter baumannii (DSMZ 30007), Acinetobacter lwoffii (DSMZ 2403, 75496), Acinetobacter radioresistens (DSMZ 6976), Acinetobacter johnsonii (DSMZ 6963), Candida albicans (ATCC 10231, 21179, 27184, 96917, 96635), Candida parapsilosis (4344).

### Example 2: Oligonucleotide probe design

Probe design and analysis were performed with the ARB software package (Ludwig et al., 2004). Selected ribosomal DNA (rDNA) sequences of pathogenic bacteria and yeasts were downloaded from the GenBank of the NCBI homepage (www.ncbi.nlm.nih.gov) and uploaded to the ARB software package to create a database comprising over 27,000 16S rDNA sequences but also over 7,000 18S rDNA sequences to detect possible mismatches with eukaryotic sequences.

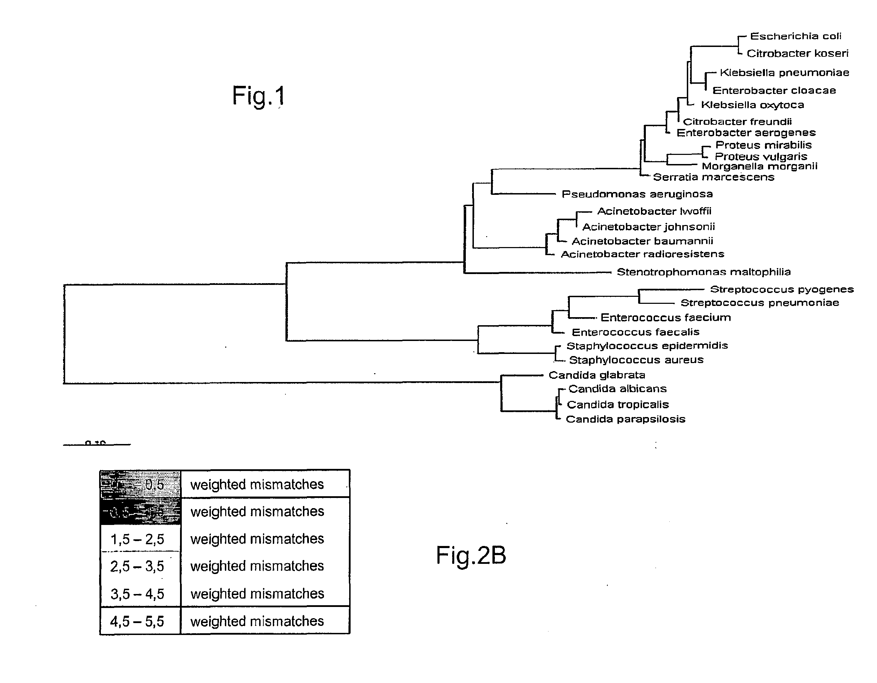

After the new sequences had been aligned to the preexisting database a phylogenetic tree was calculated using the neighbour joining method (see Fig. 1).

Probes were designed for species and selected genera based on the results of the ARB software using the Probe Design function including alterable parameter settings such as probe length (20 bases), maximum non group hits, G+C content, melting temperature and minimum hairpin loops.

Probe sequences were tested for duplex and hairpin formation and melting temperature with the software "Oligo". In their melting temperatures at first hand not matching sequences were varied by deleting or adding bases.

Final probe sequences were checked with the Probe Match function in ARB. Each generated hybridization table with sequences of organisms matching to any single probe served as input for CalcOligo (www.calcoligo.org), a software for weighted mismatch calculation. Mismatches were weighted according to experimentally determined formulas (see table 1 and table 2).

**Table 1: Weights for mismatches related to their position in the sequence. A single mismatch at the first position is weighted with 0,3 whereas mismatches at central positions were weighted highest with 1,2.**

| 5'→3' Position | | | | | | | |
|---|---|---|---|---|---|---|---|
| 1 | 2 | 3 | 4 | N | 3 | 2 | 1 |
| 0.3 | 0.6 | 1.0 | 1.2 | 1.2 | 1.1 | 0.8 | 0.3 |

**Table 2: Weights of mismatches due to the type of mismatched bases. A mismatch of adenine on the probe with cytosine on the target sequence is mismatched with 0.4, whereas a mismatch of the same probe with a guanine in the target sequence is weighted with 1.2.**

| **Probe** | **Target** | **Probe** | **Target** | **Probe** | **Target** | **Probe** | **Target** |
|---|---|---|---|---|---|---|---|
| A | - A 1.0 | G | - A 1.0 | C | - A 0.7 | T | - C 1.0 |
| | - C 0.4 | | - G 1.0 | | - C 1.0 | | - G 1.0 |
| | - G 1.2 | | - T 1.0 | | - T 1.0 | | - T 1.0 |

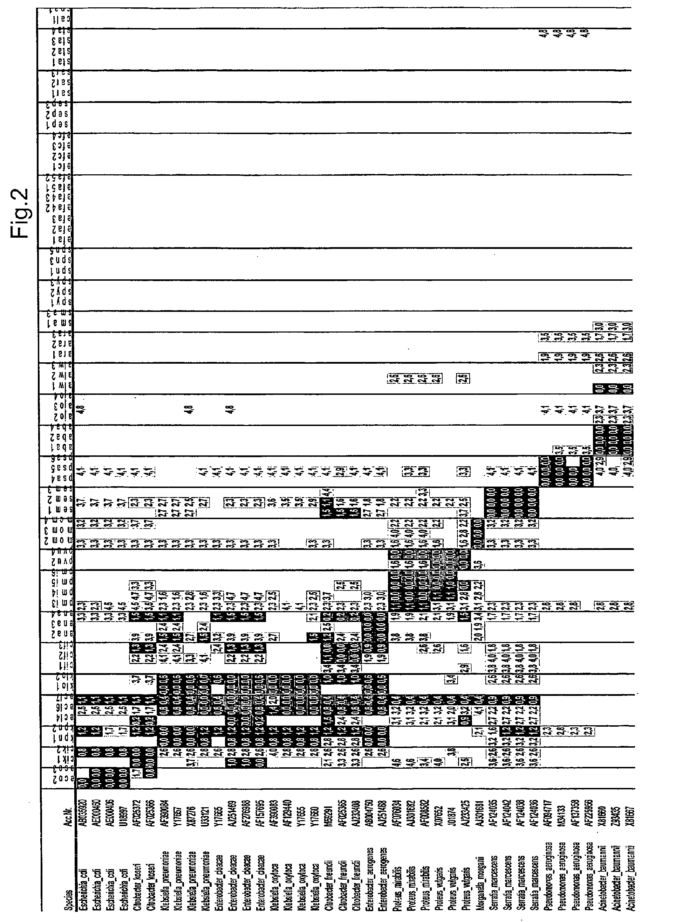

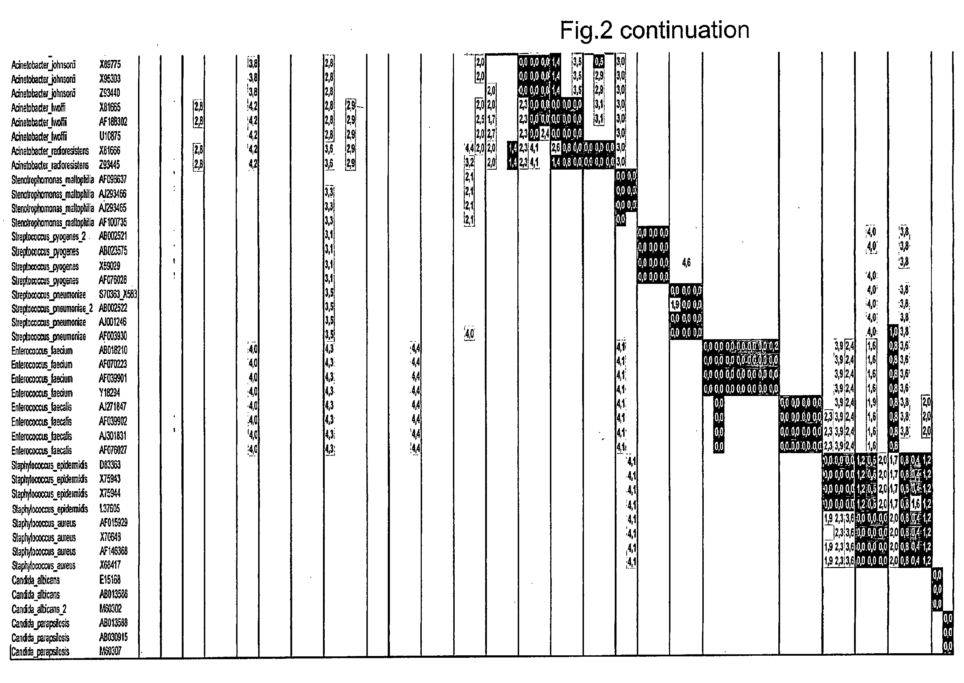

Single mismatches of each probe were added to yield a total weighted value for each species. Values were arranged to generate a hybridization matrix, sequentially tabulated in a spreadsheet (see Fig. 2 for final result of this hybridization matrix).

Due to the clinical relevance of Candida sp. they were also considered for detection, exceptionally with their 18S rRNA sequence. The tree (see Fig. 1) further shows a clear differentiation of gram positive cocci sp. and gram negative bacteria. Members of the Enterobacteriaceae familiy form an isolated group on top of the tree, indicating little relationship to the other species and strong internal sequence similarities. Within this group, the single species are closely related to each other, making the adequate identification of bacteria belonging to this group relatively difficult.

### Probe sequences

Probes were designed for selected species based on several individual sequences, selected in the ARB database. All in all 96 different DNA probes were designed using the arb software package. Additional probes were downloaded from the probeBase website (www.microbial-ecology.net/probebase/) (Loy A. et al., 2003). rDNA probes used in this study are listed in tables 3 and 4.

**Table 3: List of probes used in this study for blood stream pathogens including their nucleotide sequences and some characteristics. Abbreviations: Ab: Acinetobacter, Cb: Citrobacter, Eb: Enterobacter, Ec: Enterococcus, E: Escherichia, K: Klebsiella, M: Morganella, P: Proteus, Pm: Pseudomonas, Sr: Serratia, Sm: Stenotrophomonas, Str: Streptococcus, Sta: Staphylococcus, C: Candida**

| ***Specificity*** | ***Name*** | ***E. coli Pos.*** | ***Sequence [5'-3']*** | ***Length (bases)*** | ***Tm (°C)*** | ***GC-cont. %*** | ***SEQ ID No.*** |
|---|---|---|---|---|---|---|---|
| *Ab. baumannii* | aba1 | 64 | CAAGC- TACCTTCCCCGCT | 19 | 60.3 | 63 | 6 |
| | aba2 | 453 | GTAACGTCCACTATCTC- TAGGTAT- TAACTAAAGTAG | 36 | 59.1 | 36 | 7 |
| | aba4 | 1132 | GCAGTATCCT- TAAAGTTCCCATCCGAAA T | 29 | 60.8 | 41 | 8 |
| *Ab. johnsonii* | ajo2 | 620 | TCCCAGTATCGAAT- GCAATTCCTAAGTT | 28 | 60.1 | 39 | 9 |
| | ajo3 | 979 | GAAAGTTCTTACTATGT- CAAGACCAGGTAAG | 31 | 58.8 | 39 | 10 |
| | ajo4 | 1114 | CTTAACCCGCTG- GCAAATAAGGAAAA | 26 | 60 | 42 | 11 |
| *Ab. lwoffii* | alw1 | 133 | GAGATGT- TGTCCCCCACTAATAGGC | 25 | 60.4 | 52 | 12 |
| | alw2 | 577 | TGACTTAATTGGC- CACCTACGCG | 23 | 61 | 52 | 13 |
| | alw3 | 637 | CCCATACTCTAGCCAAC- CAGTATCG | 25 | 59.9 | 52 | 14 |
| *Ab. radioresistens* | ara1 | 78 | CGCT- GAATCCAGTAGCAAGC- TAC | 23 | 59.1 | 52 | 15 |
| | ara2 | 450 | GTCCACTATCCTAAAGTA TTAATCTAGGTAGCCT | 34 | 60.3 | 38 | 16 |
| | ara3 | 1115 | CCGAAGTGCTG- GCAAATAAGGAAA | 24 | 59.8 | 46 | 17 |
| *Cb. freundii* | cif1 | 62 | GCTCCTCTGCTAC- CGTTCG | 19 | 58.2 | 63 | 18 |
| | cif2 | 442 | CCACAACGCCTTCCTCCT CG | 20 | 61.1 | 65 | 19 |
| | cif3 | 472 | TCTGCGAGTAACGT- CAATCGCTG | 23 | 60.7 | 52 | 20 |
| *Cb. koseri* | cik1 | 469 | CGGGTAACGTCAAT- TGCTGTGG | 22 | 59.9 | 55 | 21 |
| | cik2 | 639 | CGAGACTCAAGCCTGC- CAGTAT | 22 | 60 | 55 | 22 |
| *Eb. cloacae* | ecl4 | 471 | GCGGGTAACGTCAAT- TGCTGC | 21 | 60.6 | 57 | 23 |
| | ecl6 | 643 | CTACAAGACTCCAGCCT- GCCA | 21 | 60 | 57 | 24 |
| | ecl7 | 652 | TACCCCCCTC- TACAAGACTCCA | 22 | 60 | 55 | 25 |
| *Eb. aerogenes* | ena2 | 444 | GGTTATTAACCT- TAACGCCTTCCTCCT | 27 | 60.2 | 44 | 26 |
| | ena3 | 453 | CAATCGCCAAGGTTAT- TAACCTTAACGC | 28 | 60.4 | 43 | 27 |
| | ena4 | 473 | TCTGCGAGTAACGT- CAATCGCC | 22 | 60.8 | 55 | 28 |
| *K. pneumoniae* | kpn1 | 61 | GCTCTCTGTGCTAC- CGCTCG | 20 | 60.7 | 65 | 29 |
| | kpn2 | 203 | GCATGAGGCCCGAAGGTC | 18 | 58.9 | 67 | 30 |
| *K. oxytoca* | klol | 81 | TCGTCACCCGA- GAGCAAGC | 19 | 60.5 | 63 | 31 |
| | klo2 | 633 | CCAGCCTGC- CAGTTTCGAATG | 21 | 60 | 57 | 32 |
| *E. coli* | eco2 | 448 | GTAACGTCAAT- GAGCAAAGGTAT- TAACTTTACTCCCTTCC | 40 | 61.9 | 40 | 33 |
| | eco3 | 994 | CCGAAGGCACATTCT- CATCTCT- GAAAACTTCCGTGGATG | 39 | 65.6 | 49 | 34 |
| *M. morganii* | mom2 | 121 | GCCATCAGGCAG- ATCCCCATAC | 22 | 60.9 | 59 | 35 |
| | mom3 | 440 | CT- TGACACCTTCCTCCCGAC T | 21 | 59.7 | 57 | 36 |
| | mom4 | 581 | CATCTGACTCAATCAAC- CGCCTG | 23 | 59.4 | 52 | 37 |
| *P. mirabilis* | pmi3 | 247 | GTCAGCCTTTAC- CCCACCTACTAG | 24 | 59.8 | 54 | 38 |
| | pmi4 | 444 | GGGTATTAACCTTAT- CACCTTCCTCCC | 27 | 60 | 48 | 39 |
| | pmi5 | 625 | CCAACCAGTTTCAGAT- GCAATTCCC | 25 | 60.4 | 48 | 40 |
| | pmi6 | 820 | GTTCAAGAC- CACAACCTCTAAATCGAC | 27 | 59.3 | 44 | 41 |
| *P. vulgaris* | pvu2 | 179 | CTGCTTTG- GTCCGTAGACGTCA | 22 | 60.3 | 55 | 42 |
| | pvu4 | 1010 | TTCCCGAAG- GCACTCCTCTATCTCTA | 26 | 61.9 | 50 | 43 |
| *Pm. aerogenes* | psa4 | 585 | GATTTCACATCCAACT- TGCTGAACCA | 26 | 59.9 | 42 | 44 |
| | psa5 | 1136 | TCTCCTTAGAGTGC- CCACCCG | 21 | 61.7 | 62 | 45 |
| | psa6 | 1245 | CGTGGTAAC- CGTCCCCCTTG | 20 | 61 | 65 | 46 |
| *Sr. marcescens* | sem1 | 62 | CTCCCCTGTGCTAC- CGCTC | 19 | 60.4 | 68 | 47 |
| | sem2 | 439 | CAC- CACCTTCCTCCTCGCTG | 20 | 60.7 | 65 | 48 |
| | sem3 | 460 | GAGTAACGTCAATTGAT- GAGCGTATTAAGC | 30 | 59.8 | 40 | 49 |
| *Sm. maltophilia* | sma1 | 713 | AGCTGCCTTCGCCATG- GATGTTC | 23 | 63.7 | 57 | 50 |
| | sma3 | 1265 | TGGGATTGGCTTAC- CGTCGC | 20 | 61 | 60 | 51 |
| *Str. pneumoniae* | spn1 | 56 | CTCCTCCTTCAGCGTTC- TACTTGC | 24 | 60.7 | 54 | 52 |
| | spn3 | 201 | GGTCCATCTGGTAGTG- ATGCAAGTG | 25 | 60.9 | 52 | 53 |
| | spn5 | 634 | TCTTGCACTCAAGT- TAAACAGTTTCCAAAG | 30 | 60.1 | 37 | 54 |
| *Str. pyogenes* | spy1 | 175 | ATTACTAACATGCGT- TAGTCTCTCTTATGCG | 31 | 60.2 | 39 | 55 |
| | spy2 | 471 | CTGGTTAGTTACCGT- CACTTGGTGG | 25 | 60.8 | 52 | 56 |
| | spy3 | 623 | TTCTCCAGTTTCCAAAGC GTACATTG | 26 | 59.6 | 42 | 57 |
| *Ec. faecium* | efa1 | 67 | CAAGCTCCGGTG- GAAAAAGAAGC | 23 | 60.3 | 52 | 58 |
| | efa2 | 208 | CATCCATCAGCGACAC- CCGA | 20 | 60.4 | 60 | 59 |
| | efa3 | 1240 | ACTTCGCAACTCGT- TGTACTTCCC | 24 | 60.8 | 50 | 60 |
| | efa4 2 | 446 | CCGTCAAGGGAT- GAACAGTTACTCT- CATCCTTGTTCTTC | 39 | 66.8 | 46 | 61 |
| | efa4 3 | 1242 | ATTAGCT- TAGCCTCGCGACTTCGCA ACTCGTTGTACTTC | 39 | 69.3 | 49 | 62 |
| | efa5 1 | 65 | CTCCGGTG- GAAAAAGAAGCGT | 21 | 59 | 52 | 63 |
| | efa5 2 | 82 | CTCCCGGTGGAGCAAG | 16 | 57 | 52 | 64 |
| *Staphylococcus* | sta1 | 995 | CTCTATCTCTAGAGCG- GTCAAAGGAT | 26 | 59 | 46 | 65 |
| | sta2 | 1137 | CAGTCAACCTAGAGTGC- CCAACT | 23 | 60 | 52 | 66 |
| | sta3 | 1237 | AGCTGCCCTTTGTAT- TGTCCATT | 23 | 59 | 44 | 67 |
| | sta4 | 1264 | ATGGGATTTGCAT- GACCTCGCG | 22 | 62 | 55 | 68 |
| *Sta. aureus* | sar1 | 186 | CCGTCTTTCACTTTTGAA CCATGC | 24 | 59 | 46 | 69 |
| | sar2 | 230 | AGCTAATGCAGCGCG- GATC | 19 | 59 | 58 | 70 |
| | sar3 | 447 | TGCACAGTTACTTACA- CATATGTTCTT | 27 | 57 | 33 | 71 |
| *Sta . epidermidis* | sep1 | 1005 | AAGGG- GAAAACTCTATCTCTA- GAGGG | 26 | 59 | 46 | 72 |
| | sep2 | 983 | GGGTCAGAGGATGT- CAAGATTTGG | 24 | 59 | 50 | 73 |
| | sep3 | 993 | ATCTCTAGAGGGGTCA- GAGGATGT | 24 | 60 | 50 | 74 |
| *Ec. faecalis* | efc1 | 84 | CCACTCCTCTTTCCAAT- TGAGTGCA | 24 | 61 | 50 | 75 |
| | efc2 | 176 | GCCATGCGGCATAAACT- GTTATGC | 24 | 61 | 50 | 76 |
| | efc3 | 193 | CCCGAAAGCGCCTTTCAC TCTT | 22 | 62 | 55 | 77 |
| | efc4 | 452 | GGACGTTCAGT- TACTAACGTCCTTG | 25 | 59 | 48 | 78 |
| *C. albicans* | call | - | CCAGCGAGTATAAGCCT- TGGCC | 22 | 61.2 | 59 | 79 |
| *C. parapsilosis* | cpa1 | - | TAGCCTTTTTGGCGAAC- CAGG | 21 | 60.6 | 52 | 80 |

**Table 4: List of probes used in this study for vaginosis including their nucleotide sequences and some characteristics:**

| ***Specificity*** | ***Name*** | ***E. coli Pos.*** | ***Sequence [5'- 3']*** | ***Length (bases)*** | ***Tm (°C)*** | ***GC (%)*** | ***SEQ ID No.*** |
|---|---|---|---|---|---|---|---|
| *Atopobium vaginae* | ava1 | 136 | CUUUGCACUGGGAUAGCCUCGGG | 23 | 61 | 60.9 | 81 |
| | ava2 | 434 | GCUUUCAGCAGGGACGAGGC | 20 | 61.2 | 65 | 82 |
| | ava3 | 837 | AGAUUAUACUUUCCGUGCCGCAGC | 24 | 59.4 | 50 | 83 |
| *Bacteroides* | bac1 | 145 | CGGGGAUAGC- CUUUCGAAAGAAAGA | 25 | 58.7 | 48 | 84 |
| | bac2 | 601 | UUGUGAAAGUUUGCGGCUCAAC- CGU | 25 | 61.1 | 48 | 85 |
| | bac3 | 1155 | GACUGCCGUCGUAAGAUGUGAGG | 23 | 59.6 | 56.5 | 86 |
| *Gardnerella* | gva1 | 153 | UCUUGGAAACGGGUG- GUAAUGCUGG | 25 | 61.1 | 52 | 87 |
| *vaginalis* | gva2 | 434 | GCUUUUGAUUGGGAGCAAGC- CUUUUG | 26 | 59.5 | 46.2 | 88 |
| | gva3 | 988 | UUGACAUGUGCCUGACGACUGCA | 22 | 61.2 | 52.2 | 89 |
| *Eb. cloacae* | ecl4 | 471 | GCGGGTAACGTCAATTGCTGC | 21 | 60.6 | 57 | 90 |
| | ecl6 | 643 | CTACAAGACTCCAGCCTGCCA | 21 | 60 | 57 | 91 |
| | ecl7 | 652 | TACCCCCCTCTACAAGACTCCA | 22 | 60 | 55 | 92 |
| *Eb. aerogenes* | ena2 | 444 | GGTTATTAACCT- TAACGCCTTCCTCCT | 27 | 60.2 | 44 | 93 |
| | ena3 | 453 | CAATCGCCAAGGTTATTAACCT- TAACGC | 28 | 60.4 | 43 | 94 |
| | ena4 | 473 | TCTGCGAGTAACGTCAATCGCC | 22 | 60.8 | 55 | 95 |
| *K. pneumoniae* | kpn1 | 61 | GCTCTCTGTGCTACCGCTCG | 20 | 60.7 | 65 | 96 |
| | kpn2 | 203 | GCATGAGGCCCGAAGGTC | 18 | 58.9 | 67 | 97 |
| *K. oxytoca* | klo1 | 81 | TCGTCACCCGAGAGCAAGC | 19 | 60.5 | 63 | 98 |
| | klo2 | 633 | CCAGCCTGCCAGTTTCGAATG | 21 | 60 | 57 | 99 |
| *E. coli* | eco2 | 448 | GTAACGTCAATGAGCAAAGGTAT- TAACTTTACTCCC | 36 | 60 | 38.9 | 100 |
| | eco3 | 994 | CCGAAGGCACATTCTCATCTCT- GAAAA | 27 | 59.2 | 48.8 | 101 |
| *Mobiluncus* | mob1 | 298 | GAGGGUGGUCGGUCGCACU | 19 | 62.3 | 68.4 | 102 |
| | mob2 | 586 | GCGUCUGUCGUGAAAGCCAGC | 21 | 61.3 | 61.9 | 103 |
| | mob3 | 821 | GGAACUAGGUGUGGGAUGCUAUC | 24 | 59 | 54.2 | 104 |
| *Pm. aerogenes* | psa4 | 585 | GATTTCACATCCAACTTGCT- | 26 | 59.9 | 42 | 105 |
| | | | GAACCA | | | | |
| | psa5 | 1136 | TCTCCTTAGAGTGCCCACCCG | 21 | 61.7 | 62 | 106 |
| | psa6 | 1245 | CGTGGTAACCGTCCCCCTTG | 20 | 61 | 65 | 107 |
| *Sr. marcescens* | sem1 | 62 | CTCCCCTGTGCTACCGCTC | 19 | 60.4 | 68 | 108 |
| | sem2 | 439 | CACCACCTTCCTCCTCGCTG | 20 | 60.7 | 65 | 109 |
| | sem3 | 460 | GAGTAACGTCAATTGAT- GAGCGTATTAAGC | 30 | 59.8 | 40 | 110 |
| *Sm. maltophilia* | sma1 | 713 | AGCTGCCTTCGCCATGGATGTTC | 23 | 63.7 | 57 | 111 |
| | sma3 | 1265 | TGGGATTGGCTTACCGTCGC | 20 | 61 | 60 | 112 |
| *S. pneumoniae* | spn1 | 56 | CTCCTCCTTCAGCGTTCTACTTGC | 24 | 60.7 | 54 | 113 |
| | spn3 | 201 | GGTCCATCTGGTAGTGAT- GCAAGTG | 25 | 60.9 | 52 | 114 |
| | spn5 | 634 | TCTTGCACTCAAGT- TAAACAGTTTCCAAAG | 30 | 60.1 | 37 | 115 |
| *Ec. faecium* | efa1 | 67 | CAAGCTCCGGTGGAAAAAGAAGC | 23 | 60.3 | 52 | 116 |
| | efa2 | 208 | CATCCATCAGCGACACCCGA | 20 | 60.4 | 60 | 117 |
| | efa3 | 1240 | ACTTCGCAACTCGTTGTACTTCCC | 24 | 60.8 | 50 | 118 |
| | efa4 2 | 446 | CCGTCAAGGGATGAACAGT- TACTCTCATCCTTGTTCTTC | 39 | 66.8 | 46 | 119 |
| | efa4 3 | 1242 | ATTAGCT- TAGCCTCGCGACTTCGCAACTCGT TGTACTTC | 39 | 69.3 | 49 | 120 |
| | efa5 1 | 65 | CTCCGGTGGAAAAAGAAGCGT | 21 | 59 | 52 | 121 |
| | efa5 2 | 82 | CTCCCGGTGGAGCAAG | 16 | 57 | 52 | 122 |
| *Staphylococcus* | sta1 | 995 | CTCTATCTCTAGAGCGGTCAAAG- GAT | 26 | 59 | 46 | 123 |
| | sta2 | 1137 | CAGTCAACCTAGAGTGCCCAACT | 23 | 60 | 52 | 124 |
| | sta3 | 1237 | AGCTGCCCTTTGTATTGTCCATT | 23 | 59 | 44 | 125 |
| | sta4 | 1264 | ATGGGATTTGCATGACCTCGCG | 22 | 62 | 55 | 126 |
| *Sta. aureus* | sar1 | 186 | CCGTCTTTCACTTTTGAACCATGC | 24 | 59 | 46 | 127 |
| | sar2 | 230 | AGCTAATGCAGCGCGGATC | 19 | 59 | 58 | 128 |
| | sar3 | 447 | TGCACAGTTACTTACACATA- TGTTCTT | 27 | 57 | 33 | 129 |
| *Sta. epidermidis* | sep 1 | 1005 | AAGGGGAAAACTCTATCTCTA- GAGGG | 26 | 59 | 46 | 130 |
| | sep 2 | 983 | GGGTCAGAGGATGTCAAGATTTGG | 24 | 59 | 50 | 131 |
| | sep 3 | 993 | ATCTCTAGAGGGGTCAGAGGATGT | 24 | 60 | 50 | 132 |
| *Ec. faecalis* | efc1 | 84 | CCACTCCTCTTTCCAATTGAGT- GCA | 24 | 61 | 50 | 133 |
| | efc2 | 176 | GCCATGCGGCATAAACTGTTATGC | 24 | 61 | 50 | 134 |
| | efc3 | 193 | CCCGAAAGCGCCTTTCACTCTT | 22 | 62 | 55 | 135 |
| | efc4 | 452 | GGACGTTCAGTTACTAACGTCCT- TG | 25 | 59 | 48 | 136 |
| *C. albicans* | call | - | CCAGCGAGTATAAGCCTTGGCC | 22 | 61.2 | 59 | 137 |
| *C. parapsilosis* | cpa1 | - | TAGCCTTTTTGGCGAACCAGG | 21 | 60.6 | 52 | 138 |

### Example 3: Microarray preparation

Oligonucleotide probes were obtained from VBC Genomics (Austria). At the 5' end of each oligo 5 thymine residues were added as spacer molecules. In order to ensure covalent linkage to the reactive aldehyde group on the microarray surface (CSS-100 Silylated Slides, Cel Associates, USA) probes were 5' amino-modified. Probes were printed at different concentrations (50 µM, 20 µM and 10 µM in 3x SSC and 1.5 M betaine monohydrate) onto the silylated glass slides by a contact arrayer (Omnigrid, GeneMachines) while the adjusted air humidity was between 55 and 60%.

6 replicates of each probe were printed per microarray. Spotting was carried out with SMP 3 pins (TeleChem, USA) leading to a spot size of 100 µm diameter.

### Example 4: Target preparation

### DNA isolation

Blood samples were taken by sterile withdrawal into a 10 ml K3E tube (BD Vacutainer Systems, UK). Bacteria were spiked into blood by adjusting the appropriate density using McFarland standard # 0,5 and transfer of the correct volume or dilution into 10 ml whole blood. For the separation of leukocytes a filtration step was performed. Bacteria passed the filter. If no filtration was performed, alternatively the following Percoll procedure was applied. For preliminary blood cell lysis 3 ml of Tris-EDTA, pH 8 (10 mM Tris, 1 mM EDTA) were added, mixed and centrifuged at 10000 g for 10 min. This step was repeated to obtain a small pellet which was resuspended in physiological NaCl and carefully transferred to the top of a Percoll (Amersham Biosciences) solution. Physical density of Percoll was adjusted to 1.05 g/cm³ according to the manufacturers instructions. The density centrifugation was carried out at 1500 g for 20 min. The supernatant was discarded and the pellet was rinsed with physiological NaCl in order to remove residual Percoll. The remaining pellet was resuspended in 50 µl of distilled water and cell lysis was done by heating the suspension to 95 °C for 15 min. The DNA suspension was obtained by centrifugation at 10000 g for 10 min and transferring the supernatant to a new tube.

### DNA amplification

The 16S rRNA gene was PCR amplified employing the forward primer 27 T7 (5'-TAATACGACTCACTATAGAGAGTTTGATCMTGGCTCAG; SEQ ID No. 1) and the reverse primer 1492 (5'-TACGGYTACCTTGTTACGACTT; SEQ ID No. 2) (VBC Genomics, Austria) (0.3 nM in PCR mixture) (Gutenberger et al., 1991). The forward primers contained the T7 promoter site (5'-TAATACGACTCACTATAG-3'; SEQ ID No. 3) at their 5' end, which enabled T7 RNA polymerase mediated in vitro transcription using the PCR products as templates for direct comparison of different labelling methods (Bodrossy et al., 2003). Candida species were identified by prior amplification of the 18S rRNA gene with the primers CanFW (5'- TCCGCAGGTTCACCTAC; SEQ ID No. 4) and CanRev (5'- CAAGTCTGGTGC CAGCA; SEQ ID No. 5) (White et al., 1990).

Bacteria in 10 ml whole blood served as target scenario for optimization of generation of full length 16S rRNA amplicons. Efficiency of the PCR was optimized with bacterial DNA isolated from 1 ml blood by varying the concentrations of different components and adding PCR enhancers. Optimal conditions for a 25 µl PCR reaction mixture were: 3 U Taq DNA polymerase (Invitrogen, California), 2.5 µl 10x PCR-buffer, 2 mM MgCl₂; 10% glycerol and 0.5% betaine.

Alternatively applied PCR Mastermixes were: 1.25U GoTaq^{®} DNA Polymerase (GoTaq^{®} Flexi DNA-Polymerase, Promega Corporation), 1mM MgCL₂, 5µl 5x GoTaq-PCR-buffer, dNTP to a final PCR-concentration of 0.5 mM each (ATP, GTP, CTP and TTP) and forward- and reverse-primer at a final PCR-concentration of each 0.3nM in PCR. An also alternatively Mastermix were: 1.25U FirePol^{®} DNA Polymerase I (Solis Biodyne), 2mM MgCL₂, 2.5µl 10x GoTaq-PCR-buffer, dNTP to a final PCR-concentration of 0.5 mM each (ATP, GTP, CTP and TTP) and forward- and reverse-primer at a final PCR-concentration of each 0.15nM in PCR.

PCR cycling included an initial denaturation step at 95°C for 5 minutes, followed by 40 cycles of 95°C for 30 sec, 55°C for 1 min, and 72°C for 1 min. Temperature cycles were terminated at 72°C for 10 min to complete partial amplicons, followed by storage at 4°C until further usage.

Successful amplification was confirmed by resolving the PCR products on a 1.5% agarose gel (SeaKem, Biozym) with ethidium bromide in TBE buffer (0.1 M Tris, 90 mM boric acid, 1 mM EDTA) (Invitrogen, UK).

Amplification products were either labelled directly or in a primer extension PCR.

For direct labelling procedures either 6 nmol Cy5-dCTP (Amersham Biosciences, UK) or 0.3 nM Cy3 5'end labelled primer per reaction mixture were used.

### Labelling

Different labelling strategies such as primer extension, in vitro transcription, biotin-streptavidin-labelling, isothermal Klenow fragment based labelling, or direct PCR labelling using 5' end labelled primer were optimized and compared. Good results could also be achieved without purification of the PCR products. The primer extension method showed a good sensitivity and specificity and was therefore used as standard method. 6 µl of PCR product were used for labelling in the primer extension reaction mix, which contained 0.9 mM forward primer 27, 1.5 U Vent (exo) polymerase (New England Biolabs, UK), 3 mM MgSO₄ and 50 µM of dATP, dGTP, dTTP, dCTP and 25 µM Cy5-dCTP. The reaction mix was cycled 25x at 95°C, 60°C and 72°C each 20 sec followed by a final extension step for 5 min at 72°C. Temperature cycles were preceded by 3 min incubation at 95°C.

### Example 5: Hybridization

Prior to hybridization the microarray slides were pretreated with blocking buffer (cyanoborohydride buffer: 20 mM Na₂H PO_{4,} 10 mM NaH₂PO₄, 200 mM NaCl, 50 mM NaBH₃CN) at room temperature for 30 minutes in order to inactivate reactive groups on the slide surface.

The hybridization mixture was adjusted to a final concentration of 4x SSC, 0.1% SDS in 24 µl of amplified and labelled DNA reaction mixture. A total volume of 22 µl was transferred to a cover slip (22 x 22 mm) and applied to the microarray surface. Hybridisation was realised at 65°C in a vapour saturated chamber for 1 h. Slides were washed in 2x SSC and 0.1% SDS for 5 minutes followed by 0.2 x SSC for 2 minutes and 0.1x SSC for 1 minute. Slides were dried by centrifugation at 900g for 2 minutes.

### Example 6: Signal detection and data analysis

Slides were scanned at a resolution of 10 µm with an Axon Genepix 4000A microarray scanner (Axon, USA) at equal laser power and sensitivity level of the photomultiplier (650 pmt) for each slide. Therefore absolute and relative signal intensities presented for independent experiments are directly comparable. Obtained images were analyzed using the Genepix software and the resulting gpr-files were used for further analysis.

### Statistical evaluation

Data analysis was done in R (www.r-project.org) using the packages limma, affy, stats and class. Datasets consisted of 241 hybridisations done on 3 different layouts of the pathogen identification microarray. The different layouts share 76 probes; these were used in the analysis. All other probes were disregarded. Each pathogen is represented by 2-5 different probes with different sequences. To increase robustness, probes were spotted 6 times on the array.

Each hybridisation was represented by one gpr file, all of which were collectively stored as RGList objects in R. Signals were normalised using quantile normalisation from the affy package. Medians of the 6 spot-replicates were used for supervised k-Nearest neighbour (k=1) classification method. The classifier was validated in a leave-one-out cross-validation approach. (KNN was performed according to Ripley (1996) "Pattern Recognition and Neural Networks", Cambridge and Venables et al. (2002), "Modern Applied Statistic with S.", 4th Ed., Springer; Quantile Normalization was performed according to Bolstad et al., Bioinformatics 19(2) (2003), 185-193.)

### Normalization

Normalization is an important aspect of all microarray experiments. Usually it requires a set of probes which are expected to give a constant signal throughout all hybridizations. In the present set of experiments this was not feasible. Therefore a quantile normalization approach was chosen, based on the assumption that each array should have a number of probes which give a positive signal (corresponding to the pathogen present in the sample) and the rest of the probes a low (or no) signal. This algorithm is a between-array normalization approach which replaces the highest signal of each array by the average of the top signals across all arrays, and then the second highest by the average of all second highest signals and so on. In the density plots this is illustrated by a shift of each density plot to match the average density across all arrays.

### Example 7: In silico Hybridization matrix

A hybridization matrix was generated with the Probe Match function in the ARB software package and the CalcOligo software. The modelled hybridization behaviour of each probe (Fig. 2) was in good agreement with real experimental data.

Cross hybridization within the Enterobacteriaceae family could be expected due to highly conserved 16S rRNA sequences of each member that led to strong clustering in the predicted hybridization matrix. Probes for other species should result specific signals. Especially Gram positive species were expected to give species-specific signals. In contrast to this, Gram negative bacteria within the Citrobacter, Enterobacter, Klebsiella group exhibited less specific hybridizations.

However, even these individual species could be finally identified by specific signal patterns resulting from multiple probes. All the other gram negative bacteria could be unambiguously differentiated at the species level. 18S rRNA probes of Candida sp. showed no non-specific signal with bacterial species and provided good discrimination between species. The predicted hybridization values could be confirmed by the experimental data.

### Example 8: Specificity

Normalized signal values of 241 hybridization experiments are summarized in Fig. 3. The observed hybridization values showed low coefficient of variation (CV) amongst the 6 replicate spots and between the different assays. The CV of all specific signals ranged from 2.4% to 64,1% for 80% of the probes. The experimental results closely correlated with the predicted hybridization behaviour from ARB and CalcOligo software (comparison with Fig. 2 reveals similar hybridization intensities). As expected from CalcOligo analysis, cross-hybridizations of individual probes occurred within the Enterobacteriaceae family especially in the group of Klebsiella-Enterobacter-Citrobacter . However, specific signal patterns could be assigned to each species enabling the identification of cultures at species level.

### Example 9: Sensitivity

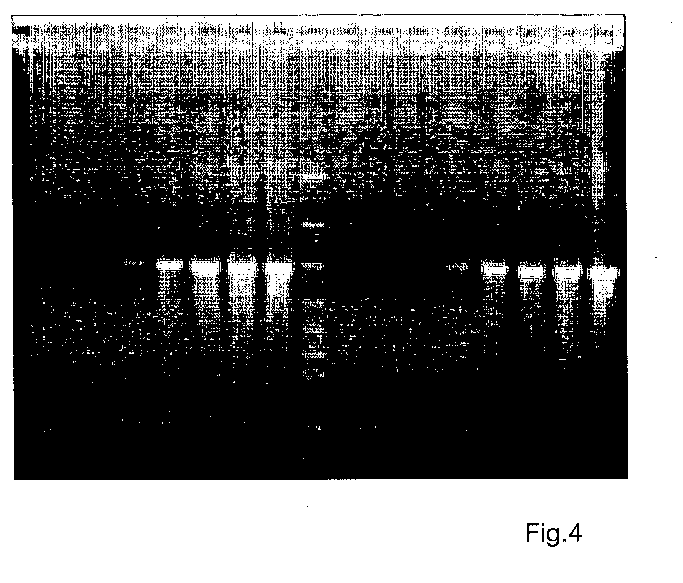

Limits of bacterial detection (LOD) were assessed with spiked blood samples and pure cultures using dilution series from 10⁸ to 10⁰ bacteria per ml from selected gram positive and gram negative bacterial species. The detection limit in pure cultures was lower than in spiked blood due to PCR interference of blood components. PCRs carried out from pure cultures were found to amplify DNA down to 103 cells per assay resulting in a clearly visible band on a 1.5% agarose gel (see Fig. 4).

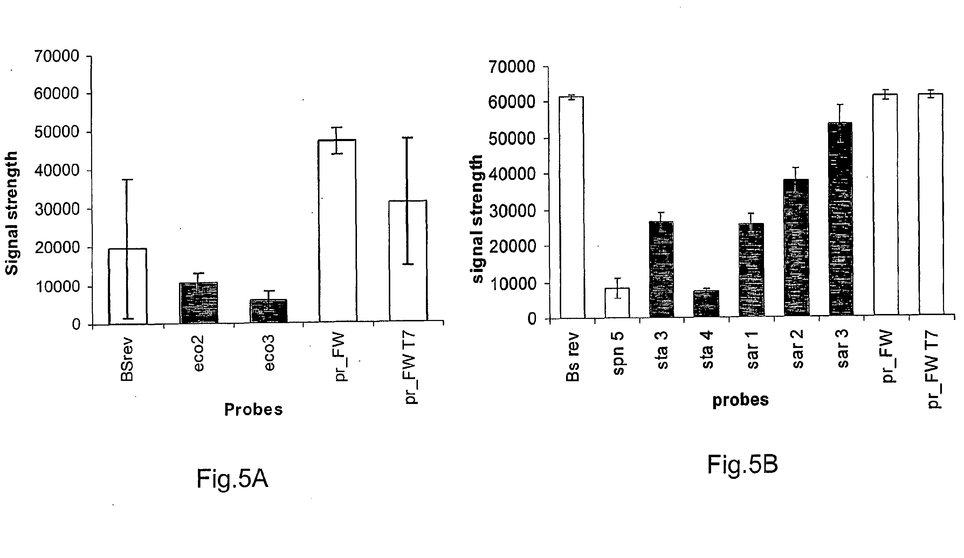

Identification based on microarrays was 100 times more sensitive than the agarose gel evaluation demonstrated. Specific and reproducible signals down to 10 bacteria per assay could be achieved for E. coli. Analysis of Staphylococcal cultures revealed the highest detection limit within the group of gram positive bacteria with about 10³ cells necessary per assay to see signals on the microarray (see Fig. 5). This difference in sensitivity can be ascribed to less efficient cell lyses due to the presence of a persistant cell wall or the presence of thermostable DNAse in Staphylococcal proteome (Heininger et al, 2004). However, the intended use of the tool demands a fast and reliable method that urges a compromise between time, applicability and sensitivity. The adoption of the protocol to different cell lyses step or an additional enzymatic treatment can further improve detection limit.

### Example 10: Parallel detection of pathogens

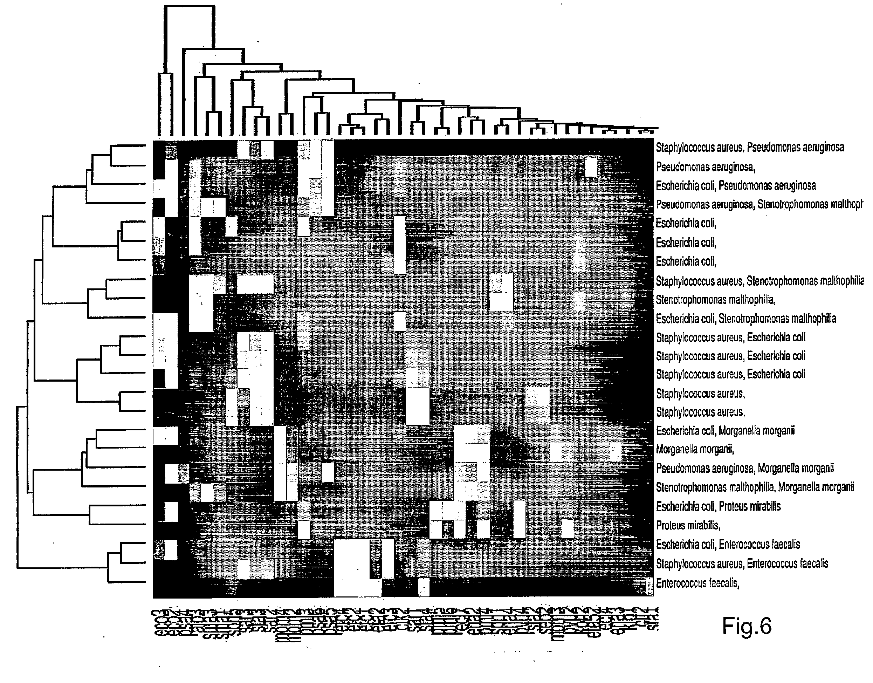

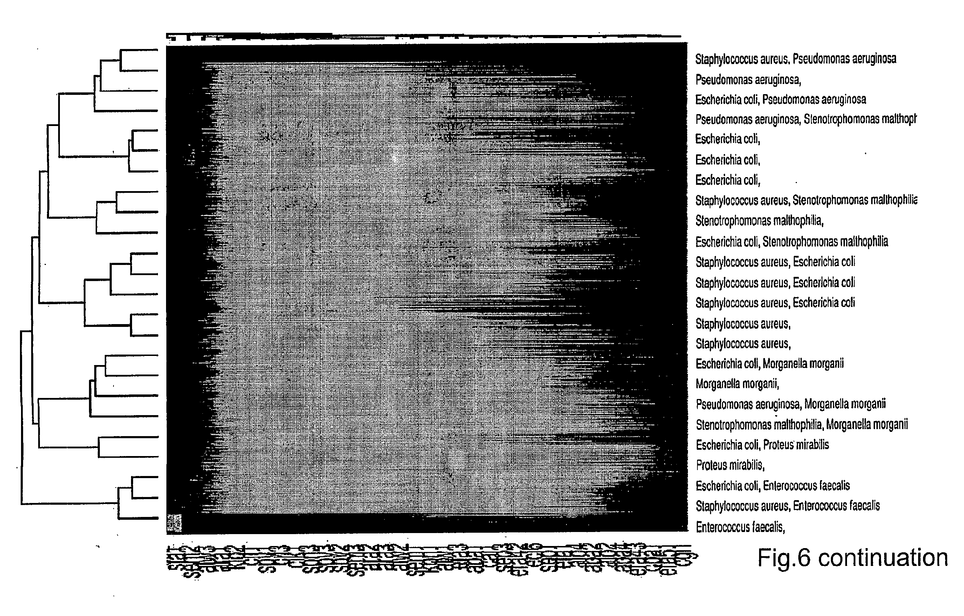

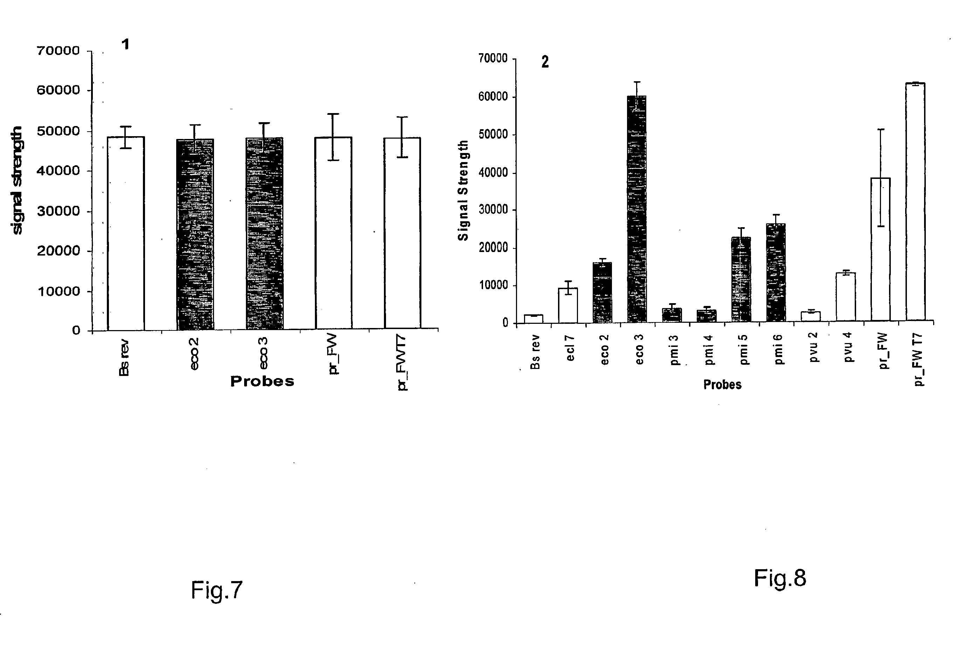

The densities of bacterial suspensions were adjusted as described in example 4 and equal amounts were added to single species and double species experiments. The hybridization results of combinations of different strains were compared to those of single strains. It was shown that at the same bacterial load the signal strengths are similar regardless of a single or a combination of species. The multiple microbial assays produced a signal pattern that displayed the compounded signals of single species hybridizations (see Fig. 6). Due to these results a clear differentiation of species in a multiple microbial infection is possible. Some experiments were carried out based on spiked blood confirming the results of pure cultures (Fig. 8)

### Example 11: Hybridization of blood sample isolates

PCR and labelling protocols were optimized with bacterial DNA isolated from blood samples to reduce interference of blood components. Addition of glycerol and betaine reduced non-specific amplification during the PCR and labelling steps in spite of large amounts of residual human DNA. By this means the yield of specific PCR product was also clearly increased resulting in equal specificities as with cultured microbes. No cross-hybridization provoked by human DNA was observed (Fig. 7). Similar results were obtained by detection of combinations of single microbes simulating multiple microbial infections as already described above. The obtained signal patterns were as specific for the added strains as those from single species microarray hybridizations (Fig. 8).

The sensitivity of the method was determined by providing a ten-fold step dilution row in 10 ml spiked blood. Detection limit was found to be as low as 10 bacteria per ml whole blood. However, as observed with pure cultures the sensitivity of gram positive bacteria is much higher, e.g. 10⁵ per ml blood for Staphylococcus aureus.

### Example 12: Candida

Four Candida sp specific probes targeting the 18S rRNA gene were included in the microbial probes present on the microarray. Fig. 3 already reveals low cross-hybridizations with bacterial target sequences indicating very high specificity of the Candida probes. Unspecific signal responses of Candida albicans targets were obtained from probes Acinetobacter lwoffi. C. parapsilosis showed low hybridization with the spn3 probe that is specific for Streptococcus pneumoniae. Protocols optimized for bacteria were also applied for Candida sp at similar sensitivity levels. In order to optimize PCR for two primer pairs, the concentration of 16S rRNA primer had to be tripled relative to the 18S rRNA primer.

### Example 13: Classification

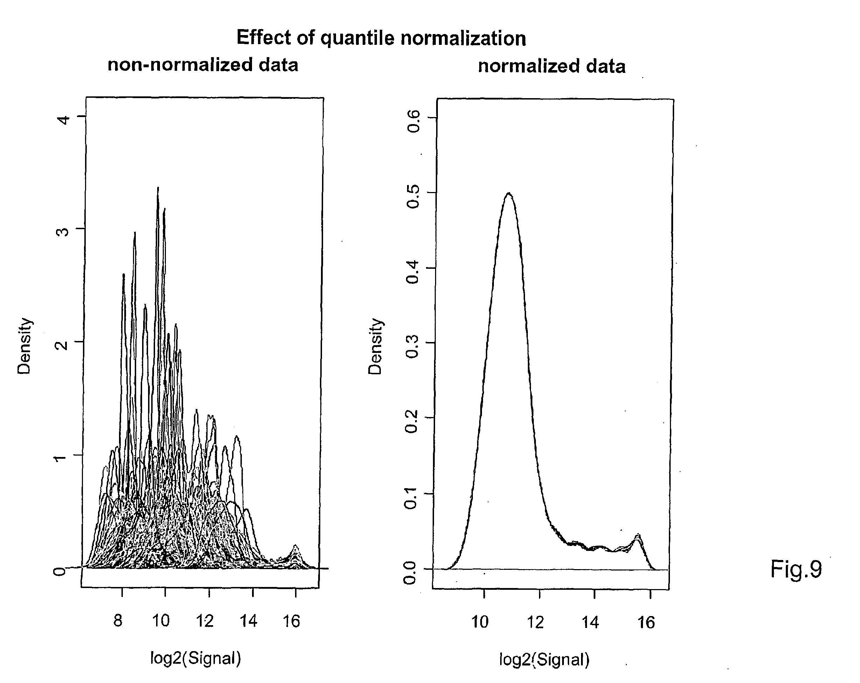

Fig. 9 shows the clear clusters of hybridizations as well as of probes. Although each probe was designed to bind to one specific pathogen, the heatmap shows that some probes are very specific to one species while others yield signals for a wider range of different organisms and a few probes do not show any specific signal at all. A classical approach would be to evaluate each probe set across all hybridizations and define a signal threshold e.g. by ROC analysis (Bilban et al., 2002) to distinguish positive from negative signals. However, since some probes show cross-hybridization between species or even genera, this would not only lead to problems with specificity, but would also mean a loss of information contained in the cross-hybridization patterns. A machine learning approach was used to classify a hybridization pattern by similarity to hybridizations with known organisms. The k-Nearest Neighbor (k=1) method was used and validated in a leave-one-out cross-validation approach. At genus level, all 241 hybridizations were stratified correctly and 96.7% at species level.

### Concluding remarks

The presented microarray for identification of blood-born pathogens is the first molecular diagnostic tool able to identify a wide range of clinically relevant bacteria and yeast directly from blood in an appreciated period of time.

The combination of PCR amplification with microarray hybridization presents a powerful tool for pathogen identification. It excels common technologies in speed while performing at an extremely high specificity. Analysis of 16S rRNA genes has been reported before to allow a more robust, reproducible, and accurate testing than phenotypic methods (Clarridge, 2004).

The arb software package analysed over 27.000 sequences, to calculate the hybridization behaviour of selected species. Predicted and experimental values showed high correlation. 23S rRNA genes were tested in parallel to the 16S rRNA targeted probes. The 16S rRNA gene was favoured over the 23S rRNA due to the larger sequence database.

Sensitivity was increased by the introduction of a DNA amplification step before the labelling. The selection of amplification and labelling strategies had a high impact on sensitivity while only causing minor changes of specificity. Hybridization to a microarray leads to about 100 times higher sensitivity compared to direct amplified target detection.

Standard clinical identification procedures require 2 days and up to 5 days for microorganisms that are difficult to cultivate. Microarray based systems enable a fast and accurate identification of microorganisms. The present protocol was carried out within 6 hours from the blood withdrawal to the presentation of results by an analysis software. Current PCR cycling times of about 2.5 hours might significantly be reduced by capillary PCR or miniaturized PCR devices allowing completion of PCR within less than 20 mins.

DNA based methods enable the detection of static or even dead cells before genome degradation e.g. in the case of administration of antibiotics when no further growth in culture can be observed (Heininger et al., 1999).

Applying a supervised k-Nearest neighbour (k=1) classification method all of the tested bacteria and yeasts were identified correctly at the genus level and 96% at the species level. High 16S rDNA sequence similarity caused misclassification in case of Proteus mirabilis and vulgaris and Acinetobacter radioresistens and baumanii.

Most published methods up to now could only recognize the affiliation to the Enterobacteriaceae family or to different gram positive genera like Staphylococcus and Streptococcus. Additionally, no technique for the simultaneous identification of bacteria and yeast was published yet (Shang et al., 2005; Jordan et al., 2005; Kempf et al., 2000; Jansen et al., 2000; Jordan and Durso, 2005).

7% of all bloodstream infections are polymicrobial (Henry et al., 1983). Signal patterns of multiple microorganisms could be interpreted from single microbial signals. Signal intensities were equal to those of single infections. The probe panel was specific for all randomly selected dual bacterial combinations. Negative controls of unspiked blood gave negative PCR amplification and hybridization results. This confirms the absence of pleomorphic bacteria or bacterial DNA in the blood of healthy humans (McLaughlin et al., 2002; Nikkari et al., 2001).

Detection levels were at 10¹ and 10³ bacteria per assay for E. coli and Staphylococcus aureus, respectively from pure cultures. The limit of detection (LOD) of other bacterial species was between 10² and 10³. Published data, suggesting higher sensitivities from pure culture, were often based on dilutions of DNA concentrates and a much smaller target sequence was amplified that only allowed the determination of bacterial presence (Wilson et al., 2002).

With spiked blood the LOD of the protocol and microarray according to the present invention was found at 10 to 10⁵ bacteria per ml blood. However, the higher LOD of spiked blood samples compared to pure cultures might result from PCR inhibitory components in blood (Al-Soud et al., 2000, 2001). Additional DNA purification can reduce the amount of these inhibitors, but high levels of residual human DNA still render lower LOD difficult.

Most identification methods based on microarray technology were published without an estimation of the LOD. Sensitivity statements for blood samples were usually based on PCR and RT-PCR experiments. LOD ranged here from 40 to 2000 CFU per ml spiked blood, although consideration of static bacteria might increase these numbers. For standard 16S rRNA PCR the LOD was at 10⁴ for E.coli and 10⁵ for Staphylococcus aureus per ml of blood. However, these assays only targeted on the confirmation of bacterial presence in blood without their identification (Jordan and Durso, 2005; Heininger et al., 2004).

Different promising approaches to increase signal strength and to further reduce the LOD of microarray analysis may be applied to this test. For example the usage of a continuously and discontinuously rotating microchamber has already been proposed (Vanderhoeven et al., 2005; Peplies et al., 2003; Liu et al., 2001; Francois et al., 2003).

A database was established serving as a classifier for the applied statistical method. Evaluation implements pattern recognition and machine learning algorithms. K-nearest-neighbour method executes an accurate identification within a fully automated platform. Moreover a software package is under development which includes the flexibility of subsequent addition of single probes, individual species, groups of species or even an exchange of the whole classifier. An enlargement of the classifier by addition of further hybridization results increases the specificity of identification, because of reduction of misinterpretation possibility due to false negative signals or cross hybridizations (especially for Proteus and Acinetobacter species). The software will allow automatic processing of gpr files from the genepix software and will retrieve genus and species names.

Additionally, recommendations of appropriate antibiotic treatments will be given from the statistical assessment of periodically updated information on antibiotic resistances.

In the present examples a rapid and sensitive method for DNA based identification of clinically relevant pathogens that cause bloodstream infections. Due to the present results this microarray is as sensitive to identify pathogens at a low concentration down to 10 bacteria per ml. Relying on the analysis of signal patterns the specificity was determined to be 100% at genus level and more than 96% at species level. This showed that an identification tool based on the 16S rRNA marker gene displays a powerful approach for routine clinical laboratory. In comparison to standard procedures, using blood cultures, a microarray identification can be performed within 6 hours and also considers multimicrobial infections. Additionally the number of identifiable organisms can easily be extended by new pathogens.

A preferred embodiment of the present invention is to provide multispecific probes which specifically identify more than 1 species within the family of Enteroceae, especially probes specifically identifying Enterobacter, Klebsiella and Citrobacter.

## Claims

1. Method for identification of microbial pathogens, preferably human pathogens, in a body fluid sample comprising the following steps:
a) providing a body fluid sample,
b) lysing the microbial pathogens and performing a nucleic acid amplification reaction, preferably PCR, on the microbial DNA encoding 16S or 18S rRNA wherein or whereafter the amplified nucleic acids are labelled,
c) contacting the labelled amplified nucleic acids of step b) with a microarray comprising on defined areas on the microarray's surface immobilised multispecific probes for microbial DNA encoding 16S or 18S rRNA from microbial pathogens,
d) detecting the binding of one or more species of the labelled amplified nucleic acids to a probe by detecting a labelled amplified nucleic acid being specifically bound to the microarray, and
e) identifying a microbial pathogen in the body fluid sample by correlating the detected binding of the labelled amplified nucleic acids with the defined areas of the immobilised multispecific probes for microbial DNA encoding 16S or 18S rRNA from microbial pathogens, comprising using the information of binding of labelled nucleic acids to multispecific probes immobilised on the microarray's surface by using predicted hybridisation patterns with weighted mismatches.

2. Method according to claim 1, **characterised in that** the nucleic acid amplification reaction on the microbial DNA encoding 16S or 18S rRNA is performed with universal primers for the microbial DNA encoding 16S or 18S rRNA, preferably with not more than eight primers, more preferred with not more than six primers, preferably with not more than four primers, in particular preferred with the primers according to Seq.ID Nos. 1, 2, 4 and 5, preferably with the GoTaq^{®} or FirePol^{®} DNA Polymerase.

3. Method according to claim 1 or 2, **characterised in that** between step a) and step b) a filtering step is performed, wherein the sample is filtered through a filter withholding leukocytes present in said body fluid sample, preferably a blood sample, but not withholding the microbial pathogens.

4. Method according to any one of claims 1 to 3, **characterised in that** the labelling of the nucleic acids is performed by primer extension, in vitro transcription, biotin-streptavidin-labelling, isothermal Klenow fragment based labelling or direct nucleic amplification labelling, preferably by direct PCR labelling.

5. Method according to any one of claims 1 to 4, **characterised in that** the amplified labelled nucleic acids are directly applied to the microarray without a purification or washing step after the nucleic acid amplification reaction.

6. Method according to any one of claims 1 to 5, **characterised in that** the microarray comprises immobilised probes for microbial DNA encoding 16S or 18S rRNA from at least ten, preferably at least 15, especially at least 20, of the following microbial pathogens: Escherichia coli, Enterobacter aerogenes, Enterobacter cloacae, Klebsiella pneumoniae, Klebsiella oxytoca , Citrobacter koseri, Citrobacter freundii, Staphylococcus aureus, Staphylococcus epidermidis, Enterococcus faecalis, Enterococcus faecium, Streptococcus pneumoniae, Streptococcus pyogenes, Proteus mirabilis, Proteus vulgaris, Serratia marcescens, Morganella morganii, Pseudomonas aeruginosa, Stenotrophomonas maltophilia, Acinetobacter baumannii, Acinetobacter lwoffii, Acinetobacter radioresistens, Acinetobacter johnsonii, Candida albicans, Candida parapsilosis, preferably comprising probes for at least one strain of at least 10 different species, preferably of at least 15 different species, especially of at least 20 different species, of the following species: Escherichia coli, Enterobacter aerogenes, Enterobacter cloacae, Klebsiella pneumoniae, Klebsiella oxytoca, Citrobacter koseri, Citrobacter freundii, Staphylococcus aureus, Staphylococcus epidermidis, Enterococcus faecalis, Enterococcus faecium, Streptococcus pneumoniae, Streptococcus pyogenes, Proteus mirabilis , Proteus vulgaris, Serratia marcescens, Morganella morganii, Pseudomonas aeruginosa, Stenotrophomonas maltophilia, Acinetobacter baumannii, Acinetobacter lwoffii, Acinetobacter radioresistens, Acinetobacter johnsonii, Candida albicans, Candida parapsilosis.

7. Method according to any one of claims 1 to 6, **characterised in that** the microarray comprises at least 10, preferably 20, more preferred at least 30, especially at least 40, multispecific immobilised probes, and/or at least 20%, preferably at least 40%, especially at least 50%, of the probes immobilised on the microarray are multispecific probes.

8. Method according to any one of claims 1 to 7, **characterised in that** the microarray comprises at least 5, preferably at least 10, more preferred at least 20, even more preferred at least 30, especially at least 50, of the probes according to Seq.ID Nos 6 to 80.

9. Method according to any one of claims 1 to 8, **characterised in that** the probes on the microarray are selected to represent at least 80%, preferably at least 90%, more preferred at least 95%, especially at least 98%, of the microbial, especially bacterial, pathogens connected with or suspected of being connected with sepsis.

10. Method according to any one of claims 1 to 9, **characterized in that** the microbial pathogen is of blood stream infections and the body fluid sample is a blood sample, or a vaginosis pathogen and the body fluid sample is a vaginal fluid sample, preferably wherein the microarray comprises at least 5, preferably at least 10, more preferred at least 20, even more preferred at least 30, especially at least 50, of the probes according to Seq.ID Nos 81 to 138.

11. Method according to claim 10, **characterised in that** the microarray comprises immobilised probes for microbial DNA encoding 16S or 18S rRNA from at least one, preferably at least 2, especially at least 3, of the following microbial pathogens: Gardnerella vaginalis, Atopobium, Mobiluncus and Bacteroides.

12. Method for identification of microbial pathogens in a body fluid sample comprising the following steps:
a) providing a body fluid sample suspected to contain such microbial pathogens,
b) lysing the microbial pathogens and performing a nucleic acid amplification reaction on the microbial DNA encoding 16S or 18S rRNA,
c) contacting the amplified nucleic acids of step b) with a microarray comprising on defined areas on the microarray's surface immobilised multispecific probes for microbial DNA encoding 16S or 18S rRNA from microbial pathogens,
d) detecting the binding of one or more species of the amplified nucleic acids to a probe by detecting a amplified nucleic acid being specifically bound to the microarray by a device of the microarray which detects the binding event of an amplified nucleic acid to an immobilised probe, and
e) identifying a microbial pathogen in the body fluid sample by correlating the detected binding of the amplified nucleic acids with the defined areas of the multispecific immobilised probes for microbial DNA encoding 16S or 18S rRNA from microbial pathogens, using the information of binding of amplified nucleic acids to multispecific probes immobilised on the microarray's surface by using predicted hybridisation patterns with weighted mismatches,
preferably wherein the specific embodiments as defined in any one of claims 2 to 11 are applied, except that instead of labelled amplified nucleic acids non-labelled nucleic acids are applied.

13. Kit comprising a microarray as defined in any one of claims 1 and 6 to 12 and a computer program product for identifying a microbial pathogen by correlating detected binding of labelled or amplified nucleic acids with defined areas of bound multispecific probes for microbial DNA encoding 16S or 18S rRNA from microbial pathogens on the microarray using the information of binding of labelled or amplified nucleic acids to multispecific probes immobilised on the microarray's surface by using predicted hybridisation patterns with weighted mismatches.

14. Test kit comprising a sample holding means for a blood sample, a kit according to claim 13 and optionally primers as defined in any one of claims 1 to 4, preferably primers being specific for amplification of microbial DNA encoding 16S or 18S rRNA of the pathogens as defined in any one of claims 1, 6, 10 or 11, and optionally a filter suitable for withholing leukocytes but not withholding microbial pathogens.

15. Use of a microarray as defined in claim 13 or a test kit according to claim 14, depending on claim 10, for the identification of microbial pathogens of bloodstream infections in a blood sample, especially for monitoring the blood of a sepsis patient or a patient being at risk of developing sepsis, or for the identification of microbial pathogens of vaginosis in a vaginal fluid sample.

## Patentansprüche

1. Verfahren zur Identifizierung von mikrobiellen Pathogenen, vorzugsweise Humanpathogenen, in einer Körperflüssigkeitsprobe, umfassend die folgenden Schritte:
a) Vorsehen einer Körperflüssigkeitsprobe,
b) Lysieren der mikrobiellen Pathogene und Durchführen einer Nukleinsäure-Amplifikationsreaktion, vorzugsweise PCR, an der für 16S- oder 18S-rRNA codierenden mikrobiellen DNA, wobei oder worauf die amplifizierten Nukleinsäuren markiert werden,
c) Kontaktieren der markierten amplifizierten Nukleinsäuren aus Schritt b) mit einem Mikroarray umfassend immobilisierte multispezifische Sonden für für 16S- oder 18S-rRNA aus mikrobiellen Pathogenen codierende mikrobielle DNA auf definierten Bereichen auf der Oberfläche des Mikroarrays,
d) Nachweisen der Bindung von einer oder mehr Art(en) der markierten amplifizierten Nukleinsäuren an eine Sonde durch Nachweisen einer markierten amplifizierten Nukleinsäure, die spezifisch an den Mikroarray gebunden ist, und
e) Identifizieren eines mikrobiellen Pathogens in der Körperflüssigkeitsprobe durch Korrelieren der nachgewiesenen Bindung der markierten amplifizierten Nukleinsäuren mit den definierten Bereichen der immobilisierten multispezifischen Sonden für für 16S- oder 18S-rRNA aus mikrobiellen Pathogenen codierende mikrobielle DNA, umfassend die Verwendung der Informationen über die Bindung von markierten Nukleinsäuren an auf der Oberfläche des Mikroarray immobilisierte multispezifische Sonden, unter Verwendung von vorausgesagten Hybridisierungsmustern mit gewichteten Nichtübereinstimmungen.

2. Verfahren nach Anspruch 1, **dadurch gekennzeichnet, dass** die Nukleinsäure-Amplifikationsreaktion an der für 16S- oder 18S-rRNA codierenden mikrobiellen DNA mit universellen Primern für die für 16S- oder 18S-rRNA codierende mikrobielle DNA, vorzugsweise mit nicht mehr als acht Primern, noch bevorzugter mit nicht mehr als sechs Primern, vorzugsweise mit nicht mehr als vier Primern, besonders bevorzugt mit den Primern gemäß Seq. ID Nr. 1, 2, 4 und 5, vorzugsweise mit der GoTaq® oder FirePol® DNA-Polymerase durchgeführt wird.

3. Verfahren nach Anspruch 1 oder 2, **dadurch gekennzeichnet, dass** zwischen Schritt a) und Schritt b) ein Filtrationsschritt durchgeführt wird, worin die Probe durch ein Filter filtriert wird, das in der Körperflüssigkeitsprobe, vorzugsweise eine Blutprobe, vorhandene Leukozyten zurückhält, die mikrobiellen Pathogene aber nicht zurückhält.

4. Verfahren nach einem der Ansprüche 1 bis 3, **dadurch gekennzeichnet, dass** das Markieren der Nukleinsäuren durch Primerverlängerung, in-vitro-Transkription, Biotin-Streptavidin-Markieren, isothermisches Markieren auf Klenow-Fragment-Basis oder direktes Nukleinsäure-Amplifikationsmarkieren, vorzugsweise durch direktes PCR-Markieren, erfolgt.

5. Verfahren nach einem der Ansprüche 1 bis 4, **dadurch gekennzeichnet, dass** die amplifizierten markierten Nukleinsäuren direkt ohne Reinigungs- oder Waschschritt nach der Nukleinsäure-Amplifikationsreaktion auf den Mikroarray aufgebracht werden.

6. Verfahren nach einem der Ansprüche 1 bis 5, **dadurch gekennzeichnet, dass** der Mikroarray immobilisierte Sonden für mikrobielle DNA umfasst, die für 16S- oder 18S-rRNA aus mindestens zehn, vorzugsweise mindestens 15, insbesondere mindestens 20, der folgenden mikrobiellen Pathogene codiert: Escherichia coli, Enterobacter aerogenes, Enterobacter cloacae, Klebsiella pneumoniae, Klebsiella oxytoca, Citrobacter koseri, Citrobacter freundii, Staphylococcus aureus, Staphylococcus epidermidis, Enterococcus faecalis, Enterococcus faecium, Streptococcus pneumoniae, Streptococcus pyogenes, Proteus mirabilis, Proteus vulgaris, Serratia marcescens, Morganella morganii, Pseudomonas aeruginosa, Stenotrophomonas maltophilia, Acinetobacter baumannii, Acinetobacter lwoffii, Acinetobacter radioresistens, Acinetobacter johnsonii, Candida albicans, Candida parapsilosis, und vorzugsweise Sonden für mindestens einen Stamm von mindestens 10 verschiedenen Arten, vorzugsweise mindestens 15 verschiedenen Arten, insbesondere mindestens 20 verschiedenen Arten, von den folgenden Arten umfasst: Escherichia coli, Enterobacter aerogenes, Enterobacter cloacae, Klebsiella pneumoniae, Klebsiella oxytoca, Citrobacter koseri, Citrobacter freundii, Staphylococcus aureus, Staphylococcus epidermidis, Enterococcus faecalis, Enterococcus faecium, Streptococcus pneumoniae, Streptococcus pyogenes, Proteus mirabilis, Proteus vulgaris, Serratia marcescens, Morganella morganii, Pseudomonas aeruginosa, Stenotrophomonas maltophilia, Acinetobacter baumannii, Acinetobacter lwoffii, Acinetobacter radioresistens, Acinetobacter johnsonii, Candida albicans, Candida parapsilosis.

7. Verfahren nach einem der Ansprüche 1 bis 6, **dadurch gekennzeichnet, dass** der Mikroarray mindestens 10, vorzugsweise mindestens 20, noch bevorzugter mindestens 30, insbesondere mindestens 40, multispezifische immobilisierte Sonden umfasst und/oder mindestens 20 %, vorzugsweise mindestens 40 %, insbesondere mindestens 50 %, der auf dem Mikroarray immobilisierten Sonden multispezifische Sonden sind.

8. Verfahren nach einem der Ansprüche 1 bis 7, **dadurch gekennzeichnet, dass** der Mikroarray mindestens 5, vorzugsweise mindestens 10, noch bevorzugter mindestens 20, noch mehr bevorzugt mindestens 30, insbesondere mindestens 50, Sonden gemäß Seq. ID Nr. 6 bis 80 umfasst.

9. Verfahren nach einem der Ansprüche 1 bis 8, **dadurch gekennzeichnet, dass** die Sonden auf dem Mikroarray so ausgewählt sind, dass sie mindestens 80 %, vorzugsweise mindestens 90 %, noch bevorzugter mindestens 95 %, insbesondere mindestens 98%, der mikrobiellen, insbesondere bakteriellen, Pathogene repräsentieren, die mit Sepsis in Verbindung stehen oder vermeintlich in Verbindung stehen.

10. Verfahren nach einem der Ansprüche 1 bis 9, **dadurch gekennzeichnet, dass** das mikrobielle Pathogen von Blutstrominfektionen ist und die Körperflüssigkeitsprobe eine Blutprobe ist, oder ein Vaginosepathogen ist und die Körperflüssigkeitsprobe eine vaginale Flüssigkeitsprobe ist, wobei der Mikroarray mindestens 5, vorzugsweise mindestens 10, noch bevorzugter mindestens 20, noch mehr bevorzugt mindestens 30, insbesondere mindestens 50, Sonden gemäß Seq. ID Nr. 81 bis 138 umfasst.

11. Verfahren nach Anspruch 10, **dadurch gekennzeichnet, dass** der Mikroarray immobilisierte Sonden für mikrobielle DNA umfasst, die für 16S- oder 18S-rRNA aus mindestens 1, vorzugsweise mindestens 2, insbesondere mindestens 3 der folgenden mikrobiellen Pathogene codiert: Gardnerella vaginalis, Atopobium, Mobiluncus und Bacteroides.

12. Verfahren zum Identifizieren von mikrobiellen Pathogenen in einer Körperflüssigkeitsprobe, umfassend die folgenden Schritte:
a) Vorsehen einer Körperflüssigkeitsprobe, die im Verdacht steht, solche mikrobiellen Pathogene zu enthalten,
b) Lysieren der mikrobiellen Pathogene und Durchführen einer Nukleinsäure-Amplifikationsreaktion an der für 16S- oder 18S-rRNA codierenden mikrobiellen DNA,
c) Kontaktieren der amplifizierten Nukleinsäuren aus Schritt b) mit einem Mikroarray umfassend immobilisierte multispezifische Sonden für für 16S- oder 18S-rRNA aus mikrobiellen Pathogenen codierende mikrobielle DNA auf definierten Bereichen auf der Oberfläche des Mikroarrays,
d) Nachweisen der Bindung von einer oder mehr Art(en) der amplifizierten Nukleinsäuren an eine Sonde durch Nachweisen einer speziell an den Mikroarray gebundenen amplifizierten Nukleinsäure mit Hilfe einer Mikroarray-Einrichtung, die das Bindungsereignis einer amplifizierten Nukleinsäure an eine immobilisierte Sonde nachweist, und
e) Identifizieren eines mikrobiellen Pathogens in der Körperflüssigkeitsprobe durch Korrelieren der nachgewiesenen Bindung der amplifizierten Nukleinsäuren mit den definierten Bereichen der multispezifischen immobilisierten Sonden für für 16S- oder 18S-rRNA aus mikrobiellen Pathogenen codierende mikrobielle DNA., unter Verwendung der Informationen über die Bindung von amplifizierten Nukleinsäuren an auf der Oberfläche des Mikroarray immobilisierte multispezifische Sonden, unter Verwendung von vorausgesagten Hybridisierungsmustern mit gewichteten Nichtübereinstimmungen,
wobei vorzugsweise die speziellen Ausführungsformen nach einem der Ansprüche 2 bis 11 mit der Ausnahme angewendet werden, dass anstelle von markierten amplifizierten Nukleinsäuren unmarkierte Nukleinsäuren verwendet werden.

13. Testset umfassend einen Mikroarray nach einem der Ansprüche 1 und 6 bis 12 und Anspruch 20 und ein Computerprogrammprodukt zur Identifizierung eines mikrobiellen Pathogens durch Korrelieren der nachgewiesenen Bindung der markierten oder amplifizierten Nukleinsäuren mit definierten Bereichen gebundener multispezifischer Sonden für für 16S- oder 18S-rRNA aus mikrobiellen Pathogenen codierende mikrobielle DNA auf dem Mikroarray, unter Verwendung der Informationen über die Bindung von markierten oder amplifizierten Nukleinsäuren an auf der Oberfläche des Mikroarray immobilisierte multispezifische Sonden, unter Verwendung von vorausgesagten Hybridisierungsmustern mit gewichteten Nichtübereinstimmungen.

14. Testset, umfassend ein Probenhalterungsmittel für eine Blutprobe, ein Set nach Anspruch 13 und gegebenenfalls Primer wie in einem der Ansprüche 1 bis 4 definiert, vorzugsweise Primer, die spezifisch für die Amplifikation von mikrobieller DNA sind, welche für 16S- und 18S-rRNA der Pathogene wie in einem der Ansprüche 1, 6, 10 oder 11 definiert codiert, und gegebenenfalls ein Filter, das zum Zurückhalten von Leukozyten, aber nicht zum Zurückhalten mikrobieller Pathogene geeignet ist.

15. Verwendung eines Mikroarrays nach Anspruch 13 oder eines Testsets nach Anspruch 14, abhängig von Anspruch 10, zur Identifizierung von mikrobiellen Pathogenen von Blutstrominfektionen in einer Blutprobe, insbesondere zur Überwachung des Bluts eines Sepsis-Patienten oder eines Sepsis-Risikopatienten, oder zur Identifizierung von mikrobiellen Pathogenen von Vaginose in einer vaginalen Flüssigkeitsprobe.

## Revendications

1. Procédé d'identification de pathogènes microbiens, de préférence de pathogènes humains, dans un échantillon de fluide corporel comprenant les étapes suivantes consistant à :
a) fournir un échantillon de fluide corporel,
b) lyser les pathogènes microbiens et réaliser une réaction d'amplification d'acide nucléique, de préférence une PCR, sur l'ADN microbien codant pour de l'ARNr 16S ou 18S, où ou après quoi les acides nucléiques amplifiés sont marqués,
c) mettre en contact les acides nucléiques amplifiés marqués de l'étape b) avec un microréseau comprenant sur des régions définies sur la surface du microréseau des sondes multispécifiques immobilisées pour l'ADN microbien codant pour de l'ARNr 16S ou 18S à partir de pathogènes microbiens,
d) détecter la liaison d'une ou de plusieurs espèces des acides nucléiques amplifiés marqués à une sonde par détection d'un acide nucléique amplifié marqué qui est lié spécifiquement au microréseau, et
e) identifier un pathogène microbien dans l'échantillon de fluide corporel par corrélation de la liaison détectée des acides nucléiques amplifiés marqués avec les régions définies des sondes multispécifiques immobilisées pour l'ADN microbien codant pour de l'ARNr 16S ou 18S à partir de pathogènes microbiens, consistant à utiliser l'information de liaison des acides nucléiques marqués aux sondes multispécifiques immobilisées sur la surface du microréseau en utilisant des schémas d'hybridation prévus avec des défauts d'appariement pondérés

2. Procédé selon la revendication 1, **caractérisé en ce que** la réaction d'amplification d'acide nucléique sur l'ADN microbien codant pour de l'ARNr 16S ou 18S est réalisée avec des amorces universelles pour l'ADN microbien codant pour de l'ARNr 16S ou 18S, de préférence avec pas plus de huit amorces, de manière davantage préférée avec pas plus de six amorces, de préférence avec pas plus de quatre amorces, d'une manière particulièrement préférée avec les amorces selon les SEQ ID No 1, 2, 4 et 5, de préférence avec l'ADN polymérase GoTaq® ou FirePol®.

3. Procédé selon la revendication 1 ou 2, **caractérisé en ce que** entre l'étape a) et l'étape b) une étape de filtration est réalisée, où l'échantillon est filtré à travers un filtre retenant les leucocytes présents dans ledit échantillon de fluide corporel, de préférence un échantillon de sang, mais ne retenant pas les pathogènes microbiens.

4. Procédé selon l'une quelconque des revendications 1 à 3, **caractérisé en ce que** le marquage des acides nucléiques est réalisé par extension d'amorce, transcription *in vitro*, marquage par biotine-streptavidine, marquage basé sur le fragment de Klenow isotherme ou marquage par amplification nucléique directe, de préférence par marquage par PCR directe.

5. Procédé selon l'une quelconque des revendications 1 à 4, **caractérisé en ce que** les acides nucléiques marqués amplifiés sont appliqués directement au microréseau sans une étape de purification ou de lavage après la réaction d'amplification d'acide nucléique.

6. Procédé selon l'une quelconque des revendications 1 à 5, **caractérisé en ce que** le microréseau comprend des sondes immobilisées pour de l'ADN microbien codant pour de l'ARNr 16S ou 18S à partir d'au moins 10, de préférence au moins 15, en particulier d'au moins 20, des pathogènes microbiens suivants : Escherichia coli, Enterobacter aerogenes, Enterobacter cloacae, Klebsiella pneumoniae, Klebsiella oxytoca, Citrobacter koseri, Citrobacter freundii, Staphylococcus aureus, Staphylococcus epidermidis, Enterococcus faecalis, Enterococcus faecium, Streptococcus pneumoniae, Streptococcus pyogenes, Proteus mirabilis, Proteus vulgaris, Serratia marcescens, Morganella morganii, Pseudomonas aeruginosa, Stenotrophomonas maltophilia, Acinetobacter baumannii, Acinetobacter lwoffii, Acinetobacter radioresistens, Acinetobacter johnsonii, Candida albicans, Candida parapsilosis, comprenant de préférence des sondes pour au moins une souche d'au moins 10 espèces différentes, de préférence d'au moins 15 espèces différentes, en particulier d'au moins 20 espèces différentes, des espèces suivantes : Escherichia coli, Enterobacter aerogenes, Enterobacter cloacae, Klebsiella pneumoniae, Klebsiella oxytoca, Citrobacter koseri, Citrobacter freundii, Staphylococcus aureus, Staphylococcus epidermidis, Enterococcus faecalis, Enterococcus faecium, Streptococcus pneumoniae, Streptococcus pyogenes, Proteus mirabilis , Proteus vulgaris, Serratia marcescens, Morganella morganii, Pseudomonas aeruginosa, Stenotrophomonas maltophilia, Acinetobacter baumannii, Acinetobacter lwoffii, Acinetobacter radioresistens, Acinetobacter johnsonii, Candida albicans, Candida parapsilosis.

7. Procédé selon l'une quelconque des revendications 1 à 6, **caractérisé en ce que** le microréseau comprend au moins 10, de préférence 20, de manière davantage préférée au moins 30, en particulier au moins 40, sondes immobilisées multispécifiques, et/ou au moins 20 %, de préférence au moins 40 %, en particulier au moins 50 %, des sondes immobilisées sur le microréseau sont des sondes multispécifiques.

8. Procédé selon l'une quelconque des revendications 1 à 7, **caractérisé en ce que** le microréseau comprend au moins 5, de préférence au moins 10, de manière davantage préférée au moins 20, de manière encore plus préférée au moins 30, en particulier au moins 50, des sondes selon les SEQ ID No 6 à 80.

9. Procédé selon l'une quelconque des revendications 1 à 8, **caractérisé en ce que** les sondes sur le microréseau sont choisies pour représenter au moins 80 %, de préférence au moins 90 %, de manière davantage préférée au moins 95 %, en particulier au moins 98 %, des pathogènes microbiens, en particulier bactériens, associés à ou supposés être associé à une sepsie.

10. Procédé selon l'une quelconque des revendications 1 à 9, **caractérisé en ce que** le pathogène microbien provient d'infections du courant sanguin et l'échantillon de fluide corporel est un échantillon de sang, ou un pathogène d'infection vaginale et l'échantillon de fluide corporel est un échantillon de fluide vaginal, de préférence où le microréseau comprend au moins 5, de préférence au moins 10, de manière davantage préférée au moins 20, de manière encore plus préférée au moins 30, en particulier au moins 50, des sondes selon les SEQ ID No 81 à 138.

11. Procédé selon la revendication 10, **caractérisé en ce que** le microréseau comprend des sondes immobilisées pour de l'ADN microbien codant pour de l'ARNr 16S ou 18S à partir d'au moins un, de préférence au moins 2, en particulier au moins 3, des pathogènes microbiens suivants: Gardnerella vaginalis, Atopobium, Mobiluncus et Bacteroides.

12. Procédé d'identification de pathogènes microbiens dans un échantillon de fluide corporel comprenant les étapes suivantes consistant à :
a) fournir un échantillon de fluide corporel supposé contenir de tels pathogènes microbiens,
b) lyser les pathogènes microbiens et réaliser une réaction d'amplification d'acide nucléique sur l'ADN microbien codant pour de l'ARNr 16S ou 18S,
c) mettre en contact les acides nucléiques amplifiés de l'étape b) avec un microréseau comprenant sur des régions définies sur la surface du microréseau des sondes multispécifiques immobilisées pour l'ADN microbien codant pour de l'ARNr 16S ou 18S à partir de pathogènes microbiens,
d) détecter la liaison d'une ou plusieurs espèces des acides nucléiques amplifiés à une sonde par détection d'un acide nucléique amplifié qui est lié spécifiquement au microréseau par un dispositif du microréseau qui détecte la présence d'une liaison d'un acide nucléique amplifié à une sonde immobilisée,
e) identifier un pathogène microbien dans l'échantillon de fluide corporel par corrélation de la liaison détectée des acides nucléiques amplifiés avec les régions définies des sondes immobilisées multispécifiques pour l'ADN microbien codant pour de l'ARNr 16S ou 18S à partir de pathogènes microbiens, en utilisant l'information de liaison des acides nucléiques amplifiés aux sondes multispécifiques immobilisées sur la surface du microréseau en utilisant des schémas d'hybridation prévus avec des mésappariements pondérés,
de préférence où les modes de réalisation spécifiques tels que définis dans l'une quelconque des revendications 2 à 11 sont appliqués, excepté que, à la place des acides nucléiques amplifiés marqués, des acides nucléiques non marqués sont appliqués.

13. Kit comprenant un microréseau tel que défini dans l'une quelconque des revendications 1 et 6 à 12 et un produit logiciel informatique pour identifier un pathogène microbien par corrélation de la liaison détectée des acides nucléiques marqués ou amplifiés avec des régions définies de sondes multispécifiques liées à l'ADN microbien codant pour de l'ARNr 16S ou 18S à partir de pathogènes microbiens sur le microréseau en utilisant l'information de liaison des acides nucléiques marqués ou amplifiés à des sondes multispécifiques immobilisées sur la surface du microréseau en utilisant des schémas d'hybridation prévus avec des mésappariement pondérés.

14. Kit d'essai comprenant un moyen de support d'échantillon de sang pour un échantillon de sang, un kit selon la revendication 13 et éventuellement des amorces telles que définies dans l'une quelconque des revendications 1 à 4, les amorces étant de préférence spécifiques pour l'amplification d'ADN microbien codant pour de l'ARNr 16S ou 18S des pathogènes tel que défini dans l'une quelconque des revendications 1, 6, 10 ou 11, et éventuellement un filtre approprié pour retenir des leucocytes mais ne retenant pas des pathogènes microbiens.

15. Utilisation d'un microréseau tel que défini dans la revendication 13 ou un kit d'essai selon la revendication 14, dépendante de la revendication 10, pour l'identification de pathogènes microbiens provenant d'infections du courant sanguin dans un échantillon de sang, en particulier pour surveiller le sang d'un patient atteint de sepsie ou d'un patient présentant un risque de développer une sepsie, ou pour l'identification de pathogènes microbiens d'une infection vaginale dans un échantillon de fluide vaginal.


### SEQUENCE LISTING

<110> Austrian Research Centers GmbH - ARC
<120> Identification of pathogens
<130> R 50322
<150> AT A 1148/2006
   <151> 2006-07-05
<160> 80
<170> PatentIn version 3.3
<210> 1
   <211> 38
   <212> DNA
   <213> Artificial
<220>
   <223> Primer
<400> 1
   taatacgact cactatagag agtttgatcm tggctcag 38
<210> 2
   <211> 22
   <212> DNA
   <213> Artificial
<220>
   <223> Primer
<400> 2
   tacggytacc ttgttacgac tt 22
<210> 3
   <211> 18
   <212> DNA
   <213> Artificial
<220>
   <223> Primer
<400> 3
   taatacgact cactatag 18
<210> 4
   <211> 17
   <212> DNA
   <213> Artificial
<220>
   <223> Primer
<400> 4
   tccgcaggtt cacctac 17
<210> 5
   <211> 17
   <212> DNA
   <213> Artificial
<220>
   <223> Primer
<400> 5
   caagtctggt gccagca 17
<210> 6
   <211> 19
   <212> DNA
   <213> Artificial
<220>
   <223> Probe
<400> 6
   caagctacct tcccccgct 19
<210> 7
   <211> 36
   <212> DNA
   <213> Artificial
<220>
   <223> Probe
<400> 7
   gtaacgtcca ctatctctag gtattaacta aagtag 36
<210> 8
   <211> 29
   <212> DNA
   <213> Artificial
<220>
   <223> Probe
<400> 8
   gcagtatcct taaagttccc atccgaaat 29
<210> 9
   <211> 28
   <212> DNA
   <213> Artificial
<220>
   <223> Probe
<400> 9
   tcccagtatc gaatgcaatt cctaagtt 28
<210> 10
   <211> 31
   <212> DNA
   <213> Artificial
<220>
   <223> Probe
<400> 10
   gaaagttctt actatgtcaa gaccaggtaa g 31
<210> 11
   <211> 26
   <212> DNA
   <213> Artificial
<220>
   <223> Probe
<400> 11
   cttaacccgc tggcaaataa ggaaaa 26
<210> 12
   <211> 25
   <212> DNA
   <213> Artificial
<220>
   <223> Probe
<400> 12
   gagatgttgt cccccactaa taggc 25
<210> 13
   <211> 23
   <212> DNA
   <213> Artificial
<220>
   <223> Probe
<400> 13
   tgacttaatt ggccacctac gcg 23
<210> 14
   <211> 25
   <212> DNA
   <213> Artificial
<220>
   <223> Probe
<400> 14
   cccatactct agccaaccag tatcg 25
<210> 15
   <211> 23
   <212> DNA
   <213> Artificial
<220>
   <223> Probe
<400> 15
   cgctgaatcc agtagcaagc tac 23
<210> 16
   <211> 34
   <212> DNA
   <213> Artificial
<220>
   <223> Probe
<400> 16
   gtccactatc ctaaagtatt aatctaggta gcct 34
<210> 17
   <211> 24
   <212> DNA
   <213> Artificial
<220>
   <223> Probe
<400> 17
   ccgaagtgct ggcaaataag gaaa 24
<210> 18
   <211> 19
   <212> DNA
   <213> Artificial
<220>
   <223> Probe
<400> 18
   gctcctctgc taccgttcg 19
<210> 19
   <211> 20
   <212> DNA
   <213> Artificial
<220>
   <223> Probe
<400> 19
   ccacaacgcc ttcctcctcg 20
<210> 20
   <211> 23
   <212> DNA
   <213> Artificial
<220>
   <223> Probe
<400> 20
   tctgcgagta acgtcaatcg ctg 23
<210> 21
   <211> 22
   <212> DNA
   <213> Artificial
<220>
   <223> Probe
<400> 21
   cgggtaacgt caattgctgt gg 22
<210> 22
   <211> 22
   <212> DNA
   <213> Artificial
<220>
   <223> Probe
<400> 22
   cgagactcaa gcctgccagt at 22
<210> 23
   <211> 21
   <212> DNA
   <213> Artificial
<220>
   <223> Probe
<400> 23
   gcgggtaacg tcaattgctg c 21
<210> 24
   <211> 21
   <212> DNA
   <213> Artificial
<220>
   <223> Probe
<400> 24
   ctacaagact ccagcctgcc a 21
<210> 25
   <211> 22
   <212> DNA
   <213> Artificial
<220>
   <223> Probe
<400> 25
   tacccccctc tacaagactc ca 22
<210> 26
   <211> 27
   <212> DNA
   <213> Artificial
<220>
   <223> Probe
<400> 26
   ggttattaac cttaacgcct tcctcct 27
<210> 27
   <211> 28
   <212> DNA
   <213> Artificial
<220>
   <223> Probe
<400> 27
   caatcgccaa ggttattaac cttaacgc 28
<210> 28
   <211> 22
   <212> DNA
   <213> Artificial
<220>
   <223> Probe
<400> 28
   tctgcgagta acgtcaatcg cc 22
<210> 29
   <211> 20
   <212> DNA
   <213> Artificial
<220>
   <223> Probe
<400> 29
   gctctctgtg ctaccgctcg 20
<210> 30
   <211> 18
   <212> DNA
   <213> Artificial
<220>
   <223> Probe
<400> 30
   gcatgaggcc cgaaggtc 18
<210> 31
   <211> 19
   <212> DNA
   <213> Artificial
<220>
   <223> Probe
<400> 31
   tcgtcacccg agagcaagc 19
<210> 32
   <211> 21
   <212> DNA
   <213> Artificial
<220>
   <223> Probe
<400> 32
   ccagcctgcc agtttcgaat g 21
<210> 33
   <211> 40
   <212> DNA
   <213> Artificial
<220>
   <223> Probe
<400> 33
   gtaacgtcaa tgagcaaagg tattaacttt actcccttcc 40
<210> 34
   <211> 39
   <212> DNA
   <213> Artificial
<220>
   <223> Probe
<400> 34
   ccgaaggcac attctcatct ctgaaaactt ccgtggatg 39
<210> 35
   <211> 22
   <212> DNA
   <213> Artificial
<220>
   <223> Probe
<400> 35
   gccatcaggc agatccccat ac 22
<210> 36
   <211> 21
   <212> DNA
   <213> Artificial
<220>
   <223> Probe
<400> 36
   cttgacacct tcctcccgac t 21
<210> 37
   <211> 23
   <212> DNA
   <213> Artificial
<220>
   <223> Probe
<400> 37
   catctgactc aatcaaccgc ctg 23
<210> 38
   <211> 24
   <212> DNA
   <213> Artificial
<220>
   <223> Probe
<400> 38
   gtcagccttt accccaccta ctag 24
<210> 39
   <211> 27
   <212> DNA
   <213> Artificial
<220>
   <223> Probe
<400> 39
   gggtattaac cttatcacct tcctccc 27
<210> 40
   <211> 25
   <212> DNA
   <213> Artificial
<220>
   <223> Probe
<400> 40
   ccaaccagtt tcagatgcaa ttccc 25
<210> 41
   <211> 27
   <212> DNA
   <213> Artificial
<220>
   <223> Probe
<400> 41
   gttcaagacc acaacctcta aatcgac 27
<210> 42
   <211> 22
   <212> DNA
   <213> Artificial
<220>
   <223> Probe
<400> 42
   ctgctttggt ccgtagacgt ca 22
<210> 43
   <211> 26
   <212> DNA
   <213> Artificial
<220>
   <223> Probe
<400> 43
   ttcccgaagg cactcctcta tctcta 26
<210> 44
   <211> 26
   <212> DNA
   <213> Artificial
<220>
   <223> Probe
<400> 44
   gatttcacat ccaacttgct gaacca 26
<210> 45
   <211> 21
   <212> DNA
   <213> Artificial
<220>
   <223> Probe
<400> 45
   tctccttaga gtgcccaccc g 21
<210> 46
   <211> 20
   <212> DNA
   <213> Artificial
<220>
   <223> Probe
<400> 46
   cgtggtaacc gtcccccttg 20
<210> 47
   <211> 19
   <212> DNA
   <213> Artificial
<220>
   <223> Probe
<400> 47
   ctcccctgtg ctaccgctc 19
<210> 48
   <211> 20
   <212> DNA
   <213> Artificial
<220>
   <223> Probe
<400> 48
   caccaccttc ctcctcgctg 20
<210> 49
   <211> 30
   <212> DNA
   <213> Artificial
<220>
   <223> Probe
<400> 49
   gagtaacgtc aattgatgag cgtattaagc 30
<210> 50
   <211> 23
   <212> DNA
   <213> Artificial
<220>
   <223> Probe
<400> 50
   agctgccttc gccatggatg ttc 23
<210> 51
   <211> 20
   <212> DNA
   <213> Artificial
<220>
   <223> Probe
<400> 51
   tgggattggc ttaccgtcgc 20
<210> 52
   <211> 24
   <212> DNA
   <213> Artificial
<220>
   <223> Probe
<400> 52
   ctcctccttc agcgttctac ttgc 24
<210> 53
   <211> 25
   <212> DNA
   <213> Artificial
<220>
   <223> Probe
<400> 53
   ggtccatctg gtagtgatgc aagtg 25
<210> 54
   <211> 30
   <212> DNA
   <213> Artificial
<220>
   <223> Probe
<400> 54
   tcttgcactc aagttaaaca gtttccaaag 30
<210> 55
   <211> 31
   <212> DNA
   <213> Artificial
<220>
   <223> Probe
<400> 55
   attactaaca tgcgttagtc tctcttatgc g 31
<210> 56
   <211> 25
   <212> DNA
   <213> Artificial
<220>
   <223> Probe
<400> 56
   ctggttagtt accgtcactt ggtgg 25
<210> 57
   <211> 26
   <212> DNA
   <213> Artificial
<220>
   <223> Probe
<400> 57
   ttctccagtt tccaaagcgt acattg 26
<210> 58
   <211> 23
   <212> DNA
   <213> Artificial
<220>
   <223> Probe
<400> 58
   caagctccgg tggaaaaaga agc 23
<210> 59
   <211> 20
   <212> DNA
   <213> Artificial
<220>
   <223> Probe
<400> 59
   catccatcag cgacacccga 20
<210> 60
   <211> 24
   <212> DNA
   <213> Artificial
<220>
   <223> Probe
<400> 60
   acttcgcaac tcgttgtact tccc 24
<210> 61
   <211> 39
   <212> DNA
   <213> Artificial
<220>
   <223> Probe
<400> 61
   ccgtcaaggg atgaacagtt actctcatcc ttgttcttc 39
<210> 62
   <211> 39
   <212> DNA
   <213> Artificial
<220>
   <223> Probe
<400> 62
   attagcttag cctcgcgact tcgcaactcg ttgtacttc 39
<210> 63
   <211> 21
   <212> DNA
   <213> Artificial
<220>
   <223> Probe
<400> 63
   ctccggtgga aaaagaagcg t 21
<210> 64
   <211> 16
   <212> DNA
   <213> Artificial
<220>
   <223> Probe
<400> 64
   ctcccggtgg agcaag 16
<210> 65
   <211> 26
   <212> DNA
   <213> Artificial
<220>
   <223> Probe
<400> 65
   ctctatctct agagcggtca aaggat 26
<210> 66
   <211> 23
   <212> DNA
   <213> Artificial
<220>
   <223> Probe
<400> 66
   cagtcaacct agagtgccca act 23
<210> 67
   <211> 23
   <212> DNA
   <213> Artificial
<220>
   <223> Probe
<400> 67
   agctgccctt tgtattgtcc att 23
<210> 68
   <211> 22
   <212> DNA
   <213> Artificial
<220>
   <223> Probe
<400> 68
   atgggatttg catgacctcg cg 22
<210> 69
   <211> 24
   <212> DNA
   <213> Artificial
<220>
   <223> Probe
<400> 69
   ccgtctttca cttttgaacc atgc 24
<210> 70
   <211> 19
   <212> DNA
   <213> Artificial
<220>
   <223> Probe
<400> 70
   agctaatgca gcgcggatc 19
<210> 71
   <211> 27
   <212> DNA
   <213> Artificial
<220>
   <223> probe
<400> 71
   tgcacagtta cttacacata tgttctt 27
<210> 72
   <211> 26
   <212> DNA
   <213> Artificial
<220>
   <223> Probe
<400> 72
   aaggggaaaa ctctatctct agaggg 26
<210> 73
   <211> 24
   <212> DNA
   <213> Artificial
<220>
   <223> Probe
<400> 73
   gggtcagagg atgtcaagat ttgg 24
<210> 74
   <211> 24
   <212> DNA
   <213> Artificial
<220>
   <223> Probe
<400> 74
   atctctagag gggtcagagg atgt 24
<210> 75
   <211> 25
   <212> DNA
   <213> Artificial
<220>
   <223> Probe
<400> 75
   ccactcctct ttccaattga gtgca 25
<210> 76
   <211> 24
   <212> DNA
   <213> Artificial
<220>
   <223> Probe
<400> 76
   gccatgcggc ataaactgtt atgc 24
<210> 77
   <211> 22
   <212> DNA
   <213> Artificial
<220>
   <223> Probe
<400> 77
   cccgaaagcg cctttcactc tt 22
<210> 78
   <211> 25
   <212> DNA
   <213> Artificial
<220>
   <223> probe
<400> 78
   ggacgttcag ttactaacgt ccttg 25
<210> 79
   <211> 22
   <212> DNA
   <213> Artificial
<220>
   <223> probe
<400> 79
   ccagcgagta taagccttgg cc 22
<210> 80
   <211> 21
   <212> DNA
   <213> Artificial
<220>
   <223> Probe
<400> 80
   tagccttttt ggcgaaccag g 21